# FASCICULE DE BREVET EUROPEEN

(11) **EP 0 750 043 B1**
(45) Date de publication et mention de la délivrance du brevet: **23.05.2001**
(21) Numéro de dépôt: 95203663.0
(22) Date de dépôt: 28.12.1995
(51) Int. Cl.: C12N 15/52, C12N 15/74, C12N 9/00, C12N 1/21, C12P 19/14, C12Q 1/68

(54) **Bactéries lactiques produisant des exopolysaccharides**
Exopolysaccharide-produzierende Milchsäurebakterien
Exopolysaccharides-producing lactic acid bacteria

(30) Priorité: 20.06.1995 EP 95201669
(43) Date de publication de la demande: 27.12.1996
(73) Titulaire: SOCIETE DES PRODUITS NESTLE S.A., 1800 Vevey (CH)
(72) Inventeur: Stingele, Francesca, CH-1018 Lausanne (CH); Mollet, Beat, CH-1074 Mollie-Margot (CH)
(74) Mandataire: Van Malderen, Joelle

(56) Documents cités:
- WO-A-88/00948
- WO-A-92/02142
- DEV. BIOL. STAND. (1995), 85(GENETICS OF STREPTOCOCCI, ENTEROCOCCI AND LACTOCOCCI), 487-493 CODEN: DVBSA3;ISSN: 0301-5149, 1995, XP000603799 STINGELE, F. ET AL: "Homologous integration and transposition to identify genes involved in the production of exopolysaccharides in Streptococcus thermophilus"
- INFECTION AND IMMUNITY, vol. 62, no. 12, WASHINGTON US, pages 5384-5396, XP002015452 ANGELO GUIDOLIN ET AL.: "Nucleotide sequence analysis of genes essential for capsular polysaccharide biosynthesis in Streptococcus pneumoniae type 19F"
- MOLECULAR AND GENERAL GENETICS, vol. 239, no. 1-2, BERLIN DE, pages 188-195, XP002015453 ERNESTO GARC A ET AL.: "Cloning and sequencing of a gene involved in the synthesis of the capsular polysaccharide of Streptococcus pneumoniae type 3"
- BIOTECHNOLOGY LETTERS, vol. 11, no. 10, pages 709-712, XP000603812 MARISA VESCOVO ET AL.: "Plasmid-encoded ropiness production in Lactobacillus casei ssp. casei"
- CARBOHYDRATE RESEARCH, vol. 198, no. 2, 1 Mai 1990, AMSTERDAM NL, pages 313-321, XP002015454 THIERRY DOCO ET AL.: "Structure of an exocellular polysaccharide produced by Streptococcus thermophilus"
- F. STINGELE AND B. MOLLET: "Disruption of the gene encoding penicillin-binding protein 2b (pbp2b) causes altered cell morphology and cease in exopolysaccharide production in Streptococcus thermophilus Sfi6", MOLECULAR MICROBIOLOGY, , -1996, Vol. 22, no. 2, pages 357 à 366

### LISTE DE SEQUENCES

(1) INFORMATIONS GENERALES:
   (i) DEPOSANT:
      (A) NOM: SOCIETE DES PRODUITS NESTLE
      (B) RUE: AVENUE NESTLE 55
      (C) VILLE: VEVEY
      (D) ETAT OU PROVINCE: CANTON DE VAUD
      (E) PAYS: SUISSE
      (F) CODE POSTAL: 1800
      (G) TELEPHONE: (41) 21 924 4760
      (H) TELECOPIE: (41) 21 924 2880
   (ii) TITRE DE L' INVENTION: BACTERIES LACTIQUES PRODUISANT DES EPS
   (iii) NOMBRE DE SEQUENCES: 19
   (iv) FORME DECHIFFRABLE PAR ORDINATEUR:
      (A) TYPE DE SUPPORT: Floppy disk
      (B) ORDINATEUR: IBM PC compatible
      (C) SYSTEME D' EXPLOITATION: PC-DOS/MS-DOS
      (D) LOGICIEL: PatentIn Release #1.0, Version #1.30 (OEB)
(2) INFORMATIONS POUR LA SEQ ID NO: 1:
   (i) CARACTERISTIQUES DE LA SEQUENCE:
      (A) LONGUEUR: 14602 paires de bases
      (B) TYPE: nucléotide
      (C) NOMBRE DE BRINS: double
      (D) CONFIGURATION: linéaire
   (ii) TYPE DE MOLECULE: ADN (g,nomique)
   (ix) CARACTERISTIQUE:
      (A) NOM/CLE: CDS
      (B) EMPLACEMENT:352..1803
      (D) AUTRES INFORMATIONS:/product= "epsA"
   (ix) CARACTERISTIQUE:
      (A) NOM/CLE: CDS
      (B) EMPLACEMENT:1807..2535
      (D) AUTRES INFORMATIONS:/product= "epsB"
   (ix) CARACTERISTIQUE:
      (A) NOM/CLE: CDS
      (B) EMPLACEMENT:2547..3239
      (D) AUTRES INFORMATIONS:/product= "epsC"
   (ix) CARACTERISTIQUE:
      (A) NOM/CLE: CDS
      (B) EMPLACEMENT:3249..3995
      (D) AUTRES INFORMATIONS:/product= "epsD"
   (ix) CARACTERISTIQUE:
      (A) NOM/CLE: CDS
      (B) EMPLACEMENT:4051..4731
      (D) AUTRES INFORMATIONS:/product= "epsE"
   (ix) CARACTERISTIQUE:
      (A) NOM/CLE: CDS
      (B) EMPLACEMENT:4898..5854
      (D) AUTRES INFORMATIONS:/product= "epsF"
   (ix) CARACTERISTIQUE:
      (A) NOM/CLE: CDS
      (B) EMPLACEMENT:6425..7540
      (D) AUTRES INFORMATIONS:/product= "epsG"
   (ix) CARACTERISTIQUE:
      (A) NOM/CLE: CDS
      (B) EMPLACEMENT:7736..8212
      (D) AUTRES INFORMATIONS:/product= "epsH"
   (ix) CARACTERISTIQUE:
      (A) NOM/CLE: CDS
      (B) EMPLACEMENT:8221..9192
      (D) AUTRES INFORMATIONS:/product= "epsI"
   (ix) CARACTERISTIQUE:
      (A) NOM/CLE: CDS
      (B) EMPLACEMENT:9285..10364
      (D) AUTRES INFORMATIONS:/product= "epsJ"
   (ix) CARACTERISTIQUE:
      (A) NOM/CLE: CDS
      (B) EMPLACEMENT:10392..11339
      (D) AUTRES INFORMATIONS:/product= "epsK"
   (ix) CARACTERISTIQUE:
      (A) NOM/CLE: misc feature
      (B) EMPLACEMENT:11302..12222
      (D) AUTRES INFORMATIONS:/product= "CDS (epsL) recouvrant le CDS aux nucleotides 10392-11339"
   (ix) CARACTERISTIQUE:
      (A) NOM/CLE: CDS
      (B) EMPLACEMENT:12233..13651
      (D) AUTRES INFORMATIONS:/product= "epsM"
   (ix) CARACTERISTIQUE:
      (A) NOM/CLE: misc_feature
      (B) EMPLACEMENT:13732..14305
      (D) AUTRES INFORMATIONS:/function= "cadre de lecture ouverte porte par le brin complementaire" /product= "orfz"
   (ix) CARACTERISTIQUE:
      (A) NOM/CLE: terminator
      (B) EMPLACEMENT:230..252
   (ix) CARACTERISTIQUE:
      (A) NOM/CLE: promoter
      (B) EMPLACEMENT:274..302
   (ix) CARACTERISTIQUE:
      (A) NOM/CLE: RBS
      (B) EMPLACEMENT:340..345
   (xi) DESCRIPTION DE LA SEQUENCE: SEQ ID NO: 1:
   TAGTTTGTAA AAGGACGCCA TTTGGTCGTC CTTTTGTGTT GTAGCTAATA TCTGTTCGAA      60
   GTGATAATAA GTTAAAATTT TTCAAACTAC TAGAAAAAAT AAAAATATTT GGAAGAAGAA     120
   GACTTATAAT AAATAGGTAA ATATCTGACA ATTTAAAGTT TAACTACTAA AAATGTAAAA     180
   GATAGTTCAC AATATAATGG AAAATGATAT AAATTAAATG ATTGATATCA TAATGAAAAA     240
   CGTTTTCTTA TTTTTTTGAA AAAAGAATGA CAATTGAAAT GAGGTTGTAT TAATGTTATA     300
   ATAATAATAA TAATGGGGAA TACCTAATTT TAATTTTTAG GAGCAATTTA T ATG AGT     357
                                                           Met Ser
                                                            1
   TCG CGT ACG AAT CGT AAG CAA AAG CAT ACG AGT AAT GGA TCG TGG GGG      405
   Ser Arg Thr Asn Arg Lys Gln Lys His Thr Ser Asn Gly Ser Trp Gly
               5                  10                  15
   ATG GTC AAC GTT GGG TTG ACC ATC CTG TAT GCT ATT TTA GCA TTG GTC      453
   Met Val Asn Val Gly Leu Thr Ile Leu Tyr Ala Ile Leu Ala Leu Val
        20                  25                  30
   TTA TTA TTC ACC ATG TTC AAT TAT AAT TTC CTA TCC TTT AGG TTT TTG      501
   Leu Leu Phe Thr Met Phe Asn Tyr Asn Phe Leu Ser Phe Arg Phe Leu
    35                  40                  45                  50
   AAC ATC ATT ATC ACC ATT GGT TTG TTG GTA GTT CTT GCT ATT AGC ATC      549
   Asn Ile Ile Ile Thr Ile Gly Leu Leu Val Val Leu Ala Ile Ser Ile
                    55                  60                  65
   TTC CTT CAG AAG ACT AAG AAA TTA CCA CTA GTG ACA ACG GTT GTA CTG      597
   Phe Leu Gln Lys Thr Lys Lys Leu Pro Leu Val Thr Thr Val Val Leu
                70                  75                  80
   GTT ATC TTC TCG CTA GTT TCT CTG GTT GGT ATT TTT GGT TTT AAA CAA      645
   Val Ile Phe Ser Leu Val Ser Leu Val Gly Ile Phe Gly Phe Lys Gln
            85                  90                  95
   ATG ATT GAC ATC ACT AAC CGT ATG AAT CAG ACA GCA GCA TTT TCT GAA      693
   Met Ile Asp Ile Thr Asn Arg Met Asn Gln Thr Ala Ala Phe Ser Glu
       100                 105                 110
   GTA GAA ATG AGC ATC GTG GTT CCT AAG GAA AGT GAC ATC AAA GAT GTG      741
   Val Glu Met Ser Ile Val Val Pro Lys Glu Ser Asp Ile Lys Asp Val
   115                 120                 125                 130
   AGC CAG CTT ACT AGC GTA CAG GCA CCT ACT AAG GTT GAT AAG AAC AAT      789
   Ser Gln Leu Thr Ser Val Gln Ala Pro Thr Lys Val Asp Lys Asn Asn
                   135                 140                 145
   ATC GAG ATC TTG ATG TCA GCT CTC AAA AAA GAT AAA AAA GTT GAT GTT      837
   Ile Glu Ile Leu Met Ser Ala Leu Lys Lys Asp Lys Lys Val Asp Val
               150                 155                 160
   AAA GTT GAT GAT GTT GCC TCA TAT CAA GAA GCT TAT GAT AAT CTC AAG      885
   Lys Val Asp Asp Val Ala Ser Tyr Gln Glu Ala Tyr Asp Asn Leu Lys
           165                 170                 175
   TCT GGC AAA TCT AAA GCT ATG GTC TTG AGT GGC TCT TAT GCT AGC CTA      933
   Ser Gly Lys Ser Lys Ala Met Val Leu Ser Gly Ser Tyr Ala Ser Leu
       180                 185                 190
   TTA GAG TCT GTC GAT AGT AAT TAT GCT TCA AAT CTA AAA ACA ATT TAT      981
   Leu Glu Ser Val Asp Ser Asn Tyr Ala Ser Asn Leu Lys Thr Ile Tyr
   195                 200                 205                 210
   ACT TAT AAA ATT AAA AAG AAG AAT AGC AAC TCT GCA AAC CAA GTA GAT     1029
   Thr Tyr Lys Ile Lys Lys Lys Asn Ser Asn Ser Ala Asn Gln Val Asp
                   215                 220                 225
   TCA AGA GTC TTC AAT ATT TAT ATT AGT GGT ATT GAT ACC TAC GGT CCG     1077
   Ser Arg Val Phe Asn Ile Tyr Ile Ser Gly Ile Asp Thr Tyr Gly Pro
               230                 235                 240
   ATT TCA ACA GTG TCA CGT TCA GAT GTC AAT ATC ATT ATG ACA GTA AAC     1125
   Ile Ser Thr Val Ser Arg Ser Asp Val Asn Ile Ile Met Thr Val Asn
           245                 250                 255
   ATG AAT ACA CAT AAG ATT CTC TTG ACG ACT ACT CCA CGT GAT GCA TAC     1173
   Met Asn Thr His Lys Ile Leu Leu Thr Thr Thr Pro Arg Asp Ala Tyr
       260                 265                 270
   GTT AAG ATT CCT GGT GGT GGG GCA GAC CAG TAT GAT AAA TTA ACC CAC     1221
   Val Lys Ile Pro Gly Gly Gly Ala Asp Gln Tyr Asp Lys Leu Thr His
   275                 280                 285                 290
   GCA GGT ATT TAT GGC GTT GAA ACA TCT GAA CAA ACT CTA GAA GAT CTT     1269
   Ala Gly Ile Tyr Gly Val Glu Thr Ser Glu Gln Thr Leu Glu Asp Leu
                   295                 300                 305
   TAT GGT ATT AAG CTT GAT TAC TAT GCA CGA ATT AAC TTC ACA TCT TTC     1317
   Tyr Gly Ile Lys Leu Asp Tyr Tyr Ala Arg Ile Asn Phe Thr Ser Phe
               310                 315                 320
   CTT AAG TTG ATT GAC CAA CTT GGT GGT GTG ACA GTC CAT AAT GAT CAA     1365
   Leu Lys Leu Ile Asp Gln Leu Gly Gly Val Thr Val His Asn Asp Gln
           325                 330                 335
   GCT TTC ACA CAA GAG AAG TTT GAT TTC CCG GTT GGA GAT ATC CAA ATG     1413
   Ala Phe Thr Gln Glu Lys Phe Asp Phe Pro Val Gly Asp Ile Gln Met
       340                 345                 350
   AAT TCA GAG CAA GCA CTT GGA TTT GTT CGT GAA CGC TAT AAT TTA GAT     1461
   Asn Ser Glu Gln Ala Leu Gly Phe Val Arg Glu Arg Tyr Asn Leu Asp
   355                 360                 365                 370
   GGC GGA GAT AAT GAC CGT GGT AAA AAC CAG GAG AAA GTT ATT TCT GCG     1509
   Gly Gly Asp Asn Asp Arg Gly Lys Asn Gln Glu Lys Val Ile Ser Ala
                   375                 380                 385
   ATT TTA AAC AAG TTG GCT TCT CTA AAA TCT GTA TCA AAC TTT ACT TCA     1557
   Ile Leu Asn Lys Leu Ala Ser Leu Lys Ser Val Ser Asn Phe Thr Ser
               390                 395                 400
   ATC GTT AAT AAT CTC CAA GAC TCT GTC CAA ACG AAT ATG TCT TTG AAT     1605
   Ile Val Asn Asn Leu Gln Asp Ser Val Gln Thr Asn Met Ser Leu Asn
           405                 410                 415
   ACC ATT AAC GCT TTG GCT AAT ACA CAA CTT GAA TCA GGT TCT AAA TTT     1653
   Thr Ile Asn Ala Leu Ala Asn Thr Gln Leu Glu Ser Gly Ser Lys Phe
       420                 425                 430
   ACG GTG ACT TCT CAA GCA GTA ACA GGT ACA GGT TCA ACC GGA CAA TTG     1701
   Thr Val Thr Ser Gln Ala Val Thr Gly Thr Gly Ser Thr Gly Gln Leu
   435                 440                 445                 450
   ATC TCT TAT GCG ATG CCA AAT TCT AGT CTT TAC ATG ATG AAA CTA GAT      1749
   Ile Ser Tyr Ala Met Pro Asn Ser Ser Leu Tyr Met Met Lys Leu Asp
                   455                 460                 465
   AAT TCG AGT GTG GAA AGT GCC TCT CAA GCT ATC AAA AAA TTG ATG GAG      1797
   Asn Ser Ser Val Glu Ser Ala Ser Gln Ala Ile Lys Lys Leu Met Glu
               470                 475                 480
   GAA AAA TAA GTG ATT GAC GTT CAC TCA CAT ATT GTT TTT GAT GTT GAT      1845
   Glu Lys     Val Ile Asp Val His Ser His Ile Val Phe Asp Val Asp
                1                   5                  10
   GAT GGT CCT GAA ACT TTA GAA GAA AGT TTA GAC CTC ATT GGT GAA AGT      1893
   Asp Gly Pro Glu Thr Leu Glu Glu Ser Leu Asp Leu Ile Gly Glu Ser
        15                  20                  25
   TAC GCC CAG GGG GTA CGT AAG ATT GTT TCA ACA TCC CAT CGT CGT AAG      1941
   Tyr Ala Gln Gly Val Arg Lys Ile Val Ser Thr Ser His Arg Arg Lys
    30                  35                  40                  45
   GGG ATG TTT GAG ACT CCA GAG GAT AAA ATT TTT GCC AAC TTT AAA AAA      1989
   Gly Met Phe Glu Thr Pro Glu Asp Lys Ile Phe Ala Asn Phe Lys Lys
                    50                  55                  60
   GTA AAA GCA GAA GCA GAA GCA CTT TAT CCA GAC TTA ACT ATT TAT TAT      2037
   Val Lys Ala Glu Ala Glu Ala Leu Tyr Pro Asp Leu Thr Ile Tyr Tyr
                65                  70                  75
   GGA GGT GAA CTT TAT TAC ACC TCA GAC ATT GTG GAG AAA CTT GAA AAG      2085
   Gly Gly Glu Leu Tyr Tyr Thr Ser Asp Ile Val Glu Lys Leu Glu Lys
            80                  85                  90
   AAT CTC ATT CCG CGC ATG CAC AAC ACT CAA TTT GCT TTA ATT GAG TTT      2133
   Asn Leu Ile Pro Arg Met His Asn Thr Gln Phe Ala Leu Ile Glu Phe
        95                 100                 105
   AGT GCT CGC ACA TCT TGG AAA GAA ATT CAT AGT GGG CTT AGT AAT GTT      2181
   Ser Ala Arg Thr Ser Trp Lys Glu Ile His Ser Gly Leu Ser Asn Val
   110                 115                 120                 125
   TTG AGA GCG GGG GTA ACG CCT ATT GTT GCT CAT ATT GAG CGC TAT GAT      2229
   Leu Arg Ala Gly Val Thr Pro Ile Val Ala His Ile Glu Arg Tyr Asp
                   130                 135                 140
   GCC CTC GAA GAA AAT GCT GAC CGT GTT CGA GAA ATC ATC AAT ATG GGC      2277
   Ala Leu Glu Glu Asn Ala Asp Arg Val Arg Glu Ile Ile Asn Met Gly
               145                 150                 155
   TGC TAT ACT CAA GTC AAT AGC TCA CAT GTC CTC AAA CCA AAG CTC TTT      2325
   Cys Tyr Thr Gln Val Asn Ser Ser His Val Leu Lys Pro Lys Leu Phe
           160                 165                 170
   GGA GAT AAA GAT AAA GTA AGA AAG AAA CGT GTT CGC TTT TTC TTG GAG      2373
   Gly Asp Lys Asp Lys Val Arg Lys Lys Arg Val Arg Phe Phe Leu Glu
       175                 180                 185
   AAA AAT TTG GTT CAT ATG GTT GCT AGC GAC ATG CAT AAT CTT GGG CCG      2421
   Lys Asn Leu Val His Met Val Ala Ser Asp Met His Asn Leu Gly Pro
   190                 195                 200                 205
   AGA CCA CCA TTT ATG AAA GAT GCT TAT GAA ATT GTT AAA AAG AAC TAC      2469
   Arg Pro Pro Phe Met Lys Asp Ala Tyr Glu Ile Val Lys Lys Asn Tyr
                   210                 215                 220
   GGC TCC AAA CGT GCT AAG AAT CTT TTT ATT GAA AAT CCC AAA ACA TTA      2517
   Gly Ser Lys Arg Ala Lys Asn Leu Phe Ile Glu Asn Pro Lys Thr Leu
               225                 230                 235
   CTA GAA AAT CAA TAT TTA TAGGAGATAT T ATG AAT CAA GAT AAC ACT AAA      2567
   Leu Glu Asn Gln Tyr Leu                Met Asn Gln Asp Asn Thr Lys
           240                              1                   5
   AGT GAT GAA ATC GAC GTA CTA GCA TTG CTA CAT AAA CTT TGG ACG AAG      2615
   Ser Asp Glu Ile Asp Val Leu Ala Leu Leu His Lys Leu Trp Thr Lys
            10                  15                  20
   AAG CTT TTG ATT CTT TTC ACA GCT TTT TAT TTC GCT GTT TTC AGT TTC        2663
   Lys Leu Leu Ile Leu Phe Thr Ala Phe Tyr Phe Ala Val Phe Ser Phe
        25                  30                  35
   TTA GGT ACT TAT TTC TTT ATC CAA CCA ACA TAT ACA TCA ACA ACG CGT        2711
   Leu Gly Thr Tyr Phe Phe Ile Gln Pro Thr Tyr Thr Ser Thr Thr Arg
    40                  45                  50                  55
   ATC TAT GTT GTT AAT CAG GCA ACA GAT AAT AAG AAT CTT TCT GCT CAA        2759
   Ile Tyr Val Val Asn Gln Ala Thr Asp Asn Lys Asn Leu Ser Ala Gln
                    60                  65                  70
   GAT TTG CAA GCT GGT ACC TAT TTG GCA AAT GAC TAT AAA GAG ATT ATT        2807
   Asp Leu Gln Ala Gly Thr Tyr Leu Ala Asn Asp Tyr Lys Glu Ile Ile
                75                  80                  85
   GCA TCA AAT GAT GTA TTA TCA GAA GTT ATT AAA GAT GAA AAA TTG AAT        2855
   Ala Ser Asn Asp Val Leu Ser Glu Val Ile Lys Asp Glu Lys Leu Asn
            90                  95                 100
   TTG AGT GAG GCA GAA CTG TCT AAA ATG GTT TCA GTT AAT ATT CCT ACT        2903
   Leu Ser Glu Ala Glu Leu Ser Lys Met Val Ser Val Asn Ile Pro Thr
       105                 110                 115
   GAT ACT CGT CTT ATT TCA ATT TCT GTT AAT GCT AAA ACT GGT CAA GAT        2951
   Asp Thr Arg Leu Ile Ser Ile Ser Val Asn Ala Lys Thr Gly Gln Asp
   120                 125                 130                 135
   GCG CAA ACA CTT GCC AAT AAG GTT CGT GAA GTT GCT TCA AAA AAA ATC        2999
   Ala Gln Thr Leu Ala Asn Lys Val Arg Glu Val Ala Ser Lys Lys Ile
                   140                 145                 150
   AAG AAG GTG ACA AAA GTT GAA GAT GTC ACA ACG CTC GAA GAA GCT AAA        3047
   Lys Lys Val Thr Lys Val Glu Asp Val Thr Thr Leu Glu Glu Ala Lys
               155                 160                 165
   TTG CCA GAG TCA CCA TCT TCA CCA AAT ATC AAA CTT AAT GTG CTT CTT        3095
   Leu Pro Glu Ser Pro Ser Ser Pro Asn Ile Lys Leu Asn Val Leu Leu
           170                 175                 180
   GGG GCA GTG CTT GGA GGA TTC CTT GCA GTG GTT GGT GTA TTG GTA CGT        3143
   Gly Ala Val Leu Gly Gly Phe Leu Ala Val Val Gly Val Leu Val Arg
       185                 190                 195
   GAA ATC CTA GAT GAT CGT GTT CGC CGT CCA GAA GAT GTG GAA GAT GCC        3191
   Glu Ile Leu Asp Asp Arg Val Arg Arg Pro Glu Asp Val Glu Asp Ala
   200                 205                 210                 215
   CTT GGA ATG ACA CTT CTT GGA ATT GTC CCT GAT ACA GAT AAA ATT TAA        3239
   Leu Gly Met Thr Leu Leu Gly Ile Val Pro Asp Thr Asp Lys Ile  *
                   220                 225                 230
   GGAGAAGAA ATG CCT TTA TTA AAG TTA GTT AAA TCA AAA GTA GAC TTT          3287
             Met Pro Leu Leu Lys Leu Val Lys Ser Lys Val Asp Phe
               1               5                  10
   GCT AAA AAG ACG GAA GAG TAT TAT AAC GCT ATT CGC ACA AAT ATT CAA        3335
   Ala Lys Lys Thr Glu Glu Tyr Tyr Asn Ala Ile Arg Thr Asn Ile Gln
        15                  20                  25
   TTT TCT GGT GCT CAG ATG AAA GTG ATT GCG ATT AGC TCT GTT GAA GCT        3383
   Phe Ser Gly Ala Gln Met Lys Val Ile Ala Ile Ser Ser Val Glu Ala
    30                  35                  40                  45
   GGT GAA GGA AAA TCA ATG ATA TCT GTT AAC TTG GCG ATT TCA TTT GCT        3431
   Gly Glu Gly Lys Ser Met Ile Ser Val Asn Leu Ala Ile Ser Phe Ala
                    50                  55                  60
   AGT GTT GGG CTC CGA ACA CTT CTG ATT GAT GCG GAA ACG CGT AAT TCT        3479
   Ser Val Gly Leu Arg Thr Leu Leu Ile Asp Ala Glu Thr Arg Asn Ser
                65                  70                  75
   GTT TTG TCA GGT ACA TTT AAA TCA AAT GAG CCT TAT AAA GGT CTT TCA        3527
   Val Leu Ser Gly Thr Phe Lys Ser Asn Glu Pro Tyr Lys Gly Leu Ser
            80                  85                  90
   AAT TTC CTT TCA GGA AAT GCC GAT CTA AAT GAA ACG ATT TGC CAA ACT        3575
   Asn Phe Leu Ser Gly Asn Ala Asp Leu Asn Glu Thr Ile Cys Gln Thr
        95                 100                 105
   GAT ATT TCT GGT TTA GAT GTT ATT GCA TCT GGT CCT GTT CCA CCT AAT      3623
   Asp Ile Ser Gly Leu Asp Val Ile Ala Ser Gly Pro Val Pro Pro Asn
   110                 115                 120                 125
   CCA ACA AGT CTT TTG CAA AAT GAT AAT TTT AGA CAT TTG ATG GAA GTT      3671
   Pro Thr Ser Leu Leu Gln Asn Asp Asn Phe Arg His Leu Met Glu Val
                   130                 135                 140
   GCT CGT AGT TGT TAT GAT TAT GTC ATC ATC GAT ACA CCA CCA GTT GGT      3719
   Ala Arg Ser Cys Tyr Asp Tyr Val Ile Ile Asp Thr Pro Pro Val Gly
               145                 150                 155
   CTG GTT ATT GAT GCA GTT ATT ATT GCC CAT CAG GCT GAT GCC AGT CTT      3767
   Leu Val Ile Asp Ala Val Ile Ile Ala His Gln Ala Asp Ala Ser Leu
           160                 165                 170
   TTG GTT ACA GAA GCT GGG AAA ATT AAA CGT CGT TTC GTA ACT AAG GCC      3815
   Leu Val Thr Glu Ala Gly Lys Ile Lys Arg Arg Phe Val Thr Lys Ala
       175                 180                 185
   GTT GAA CAA TTG GTA GAA AGT GGT TCT CAG TTC TTA GGG GTC GTC CTT      3863
   Val Glu Gln Leu Val Glu Ser Gly Ser Gln Phe Leu Gly Val Val Leu
   190                 195                 200                 205
   AAT AAA GTT GAC ATG ACA GTT GAT AAA TAT GGA TTT TAT GGT TCT TAC      3911
   Asn Lys Val Asp Met Thr Val Asp Lys Tyr Gly Phe Tyr Gly Ser Tyr
                   210                 215                 220
   GGA TCA TAT GGC GAG TAT GGA AAA AAA TCT GAC CAA AAA GAA GGT CAT      3959
   Gly Ser Tyr Gly Glu Tyr Gly Lys Lys Ser Asp Gln Lys Glu Gly His
               225                 230                 235
   TCA AGA GCA CAT CGT CGT AGA AAA GTC GGT TGG AAT TAACGCGTTA           4005
   Ser Arg Ala His Arg Arg Arg Lys Val Gly Trp Asn
           240                 245
   GTGTGTTTTA AGATGTCGTT GGGAACGACA AGTGGAGGGA ATGAG ATG TCA CAA         4059
                                                     Met Ser Gln
                                                       1
   GCT AAA GAG GAA ATT TCA GAT GTT ATG ACT TAT TCA GAG CTA ACA AGT      4107
   Ala Lys Glu Glu Ile Ser Asp Val Met Thr Tyr Ser Glu Leu Thr Ser
       5                   10                  15
   CAT AAG CCC AAA ATT ATT TAT AGC TTG ATT AAG CGG ATT GGT GAT ATT      4155
   His Lys Pro Lys Ile Ile Tyr Ser Leu Ile Lys Arg Ile Gly Asp Ile
   20                  25                  30                  35
   TTG GTT AGT TCT ATT GGT TTA ATT ATT TTG ATA CCG CTA TTT TTG ATA      4203
   Leu Val Ser Ser Ile Gly Leu Ile Ile Leu Ile Pro Leu Phe Leu Ile
                   40                  45                  50
   GTT GCT TTG ATC ATG AAA TGC TCT GAA CCA ACA GCA CCT ATA TTT TTC      4251
   Val Ala Leu Ile Met Lys Cys Ser Glu Pro Thr Ala Pro Ile Phe Phe
               55                  60                  65
   TCA CAT ATT AGA AAT GGT AAA AAT GGC AAA AAG TTC AAA ATG TAT AAA      4299
   Ser His Ile Arg Asn Gly Lys Asn Gly Lys Lys Phe Lys Met Tyr Lys
           70                  75                  80
   TTT AGA ACC ATG TGT CAG GAC GCA GAA TCG ATT TTG ATG AAA GAT ACG      4347
   Phe Arg Thr Met Cys Gln Asp Ala Glu Ser Ile Leu Met Lys Asp Thr
       85                  90                  95
   GAA CTT TTT GCA AAA TTT AAG GCA AAT GGT TAT AAA CTT GAA ACG CAT      4395
   Glu Leu Phe Ala Lys Phe Lys Ala Asn Gly Tyr Lys Leu Glu Thr His
   100                 105                 110                 115
   GAA GAT CCT AGA ATT ACA AAA ATC GGT GGC ATA TTA AGG AAA ACA AGT      4443
   Glu Asp Pro Arg Ile Thr Lys Ile Gly Gly Ile Leu Arg Lys Thr Ser
                   120                 125                 130
   ATT GAT GAA TTG CCA CAA CTG ATT AAT GTT TTT TTA GGA CAA ATG TCA      4491
   Ile Asp Glu Leu Pro Gln Leu Ile Asn Val Phe Leu Gly Gln Met Ser
               135                 140                 145
   TTA GTG GGT CCA CGT CCA CTA CCA GAT AGA GAA ATC ATT GAA TAC GGT      4539
   Leu Val Gly Pro Arg Pro Leu Pro Asp Arg Glu Ile Ile Glu Tyr Gly
           150                 155                 160
   GAT AAC CAA GAA AAA TTT TTA AGC GTT AAA CCA GGC ATG ACA GGA TGG      4587
   Asp Asn Gln Glu Lys Phe Leu Ser Val Lys Pro Gly Met Thr Gly Trp
       165                 170                 175
   TGG CAA GTT TCA GGG AGA AGT ACT ATT GGG TAT CCT GAG CGG TGT CAT      4635
   Trp Gln Val Ser Gly Arg Ser Thr Ile Gly Tyr Pro Glu Arg Cys His
   180                 185                 190                 195
   CTT GAG CTT TAT TAT GTA GAA AAG TGT TGT TTT ACT TTC GAT GTT CTT      4683
   Leu Glu Leu Tyr Tyr Val Glu Lys Cys Cys Phe Thr Phe Asp Val Leu
                   200                 205                 210
   ATA TTA CTT AAG ACA ATT GGG ATT GTT TTG AAG AGA GTT GGA GCG CGT      4731
   Ile Leu Leu Lys Thr Ile Gly Ile Val Leu Lys Arg Val Gly Ala Arg
               215                 220                 225
   TAGTACTGAT GAAACAAAAA TTATTATTGA TAATAGAAGC GATGAGTGGT GGAGCCGGTC    4791
   GTCATGTACA AGACTTGATT AGTCATCTAC CTCAAGAAAA ATTTGATATT TATGTGATTT    4851
   ATTCAAATCA TAGAACAAAT CCTGTTTTTT GGAAAAAATA GTAACG ATG AAT GAG       4906
                                                      Met Asn Glu
                                                        1
   CAA GTA ACT TTT ATT TTA TGT GAT TTT CTC GTA AGA GAA ATT AAA CCG      4954
   Gln Val Thr Phe Ile Leu Cys Asp Phe Leu Val Arg Glu Ile Lys Pro
       5                   10                  15
   AAA TAT GAT TTG CTT GCT TAT CAA TTT ATT TCT AAA AAG ATT AAA GAA      5002
   Lys Tyr Asp Leu Leu Ala Tyr Gln Phe Ile Ser Lys Lys Ile Lys Glu
   20                  25                  30                  35
   ATC AAA CCA GAT ATT GTA CAT TGT CAC AGT TCA AAA GCT GGT GTT ATT      5050
   Ile Lys Pro Asp Ile Val His Cys His Ser Ser Lys Ala Gly Val Ile
                   40                  45                  50
   GGT CGT TTA GCT GCC AAA AGA CGA GGT GTT AAA AAA ATA TTT TAT ACG      5098
   Gly Arg Leu Ala Ala Lys Arg Arg Gly Val Lys Lys Ile Phe Tyr Thr
               55                  60                  65
   CCA CAT GCT TAT TCG TTT TTG GCA CCT GAA TTT AGT GGG AAG AAA AAG      5146
   Pro His Ala Tyr Ser Phe Leu Ala Pro Glu Phe Ser Gly Lys Lys Lys
           70                  75                  80
   TTT CTA TTT GTT CAA ATT GAA AAG TTT TTA AGC CGA TTT GCG ACA ACT      5194
   Phe Leu Phe Val Gln Ile Glu Lys Phe Leu Ser Arg Phe Ala Thr Thr
       85                  90                  95
   AAG ATA TTT TGT GTG TCA ATA GCG GAA ATG CAA GCT GCT CTT GAA GTA      5242
   Lys Ile Phe Cys Val Ser Ile Ala Glu Met Gln Ala Ala Leu Glu Val
   100                 105                 110                 115
   AAT CTA GAT AAA ACC GAT AAG TTT CAG GTA ATT TAT AAT GGT TTG CCA      5290
   Asn Leu Asp Lys Thr Asp Lys Phe Gln Val Ile Tyr Asn Gly Leu Pro
                   120                 125                 130
   GAA ATT GAT TTA CCA AGC AAA GAA ACG ATT CGG GCG CAA TTA GGA CTG      5338
   Glu Ile Asp Leu Pro Ser Lys Glu Thr Ile Arg Ala Gln Leu Gly Leu
               135                 140                 145
   GAA AAG GCA GCA GTT GTT ATA GGC AAT AAT GCA AAA ATG TCG GAA CAG      5386
   Glu Lys Ala Ala Val Val Ile Gly Asn Asn Ala Lys Met Ser Glu Gln
           150                 155                 160
   AAA AAT CCT ATG TTT TTT ATG GAA ATT GCC CGA AAA ATG ATT AGA CAA      5434
   Lys Asn Pro Met Phe Phe Met Glu Ile Ala Arg Lys Met Ile Arg Gln
       165                 170                 175
   AAC GCA AAT TGG CAT TTT GTG TGG GTA GGT GAT GGT CAG CTG ATG CCA      5482
   Asn Ala Asn Trp His Phe Val Trp Val Gly Asp Gly Gln Leu Met Pro
   180                 185                 190                 195
   CTT TTT CAA TCA TTT ATT AAG CAA AAT GGA CTA GAG GGA AAT ATC CAT        5530
   Leu Phe Gln Ser Phe Ile Lys Gln Asn Gly Leu Glu Gly Asn Ile His
                   200                 205                 210
   TTG CTT GGC GAG CGT CCT GAT AGT GAA ATA GTT GTG ACA GCC TAT GAC        5578
   Leu Leu Gly Glu Arg Pro Asp Ser Glu Ile Val Val Thr Ala Tyr Asp
               215                 220                 225
   ATC TTC TTG ACG ACT TCC CAA TAT GAA GGT TTA CCT TAT GCA CCA ATT        5626
   Ile Phe Leu Thr Thr Ser Gln Tyr Glu Gly Leu Pro Tyr Ala Pro Ile
           230                 235                 240
   GAA GCG ATG CGA GCT GGT GTC CCG ATT CTT GCG ACA AAA GTT GTT GGC        5674
   Glu Ala Met Arg Ala Gly Val Pro Ile Leu Ala Thr Lys Val Val Gly
       245                 250                 255
   AAT AGT GAG CTT GTG ATA GAG GGC AAA AAT GGT TAT TTG ATT GAC TTA        5722
   Asn Ser Glu Leu Val Ile Glu Gly Lys Asn Gly Tyr Leu Ile Asp Leu
   260                 265                 270                 275
   GAG TGG TCA AAA TCT GTC GAA GAA AAA TTA TAT AAG GCA GCG AAA ATA        5770
   Glu Trp Ser Lys Ser Val Glu Glu Lys Leu Tyr Lys Ala Ala Lys Ile
                   280                 285                 290
   GAT GCA CAA ATG ATT AAA GCA GAT TTT AGG CAA AGG TTT GCG ATT GAT        5818
   Asp Ala Gln Met Ile Lys Ala Asp Phe Arg Gln Arg Phe Ala Ile Asp
               295                 300                 305
   CAG ATA TTA AAG CAA ATT GAA ACA ATT TAT TTA GCT TGAATGAAGA             5864
   Gln Ile Leu Lys Gln Ile Glu Thr Ile Tyr Leu Ala
           310                 315
   ATGAGGAGGC ATAAATGCTG ATTTTGAAAT TAAAATTTCA TCTTAATTGG TACACAAACG       5924
   AAAACCATTA TTACACGTGA GTATTCGAAG ACCTGGAAAC GAGGCGATGA GCCGTATTAT       5984
   CCAGTGAACA ATGATCGTAA CAACAAACTC TATACTGCCT ATAAGCGTCT TGCCGAGCAA       6044
   CAAGAGAATG TCATTTTCGG TGGACGTCTA GGTCACTACC GTTACTACGA TATGCACCAG       6104
   GTAATTGGAG CTGCCTTGCA GTGTGTCAGA AATGAAGTGA AGTAAATCTT GATGAAGTTG       6164
   AATAACTTTA AGTAATTTTA TACTTAATCC AATTGATGAA AATATTTTTG TATCGATTTA       6224
   TCTTCTGTAA GAAGAGTCCT AATCGTTTAA AAAATGTACA ATTGAGTTTT TATATTTTTA       6284
   AATAAAGTTA CTTTTAAGTC GTGTTATAGA ATATACATGA ATAGGTGTAT TAGAAAATTT       6344
   ATTAATCTAA TCCTCGAAAA TAACTGACTG TAAGGAATCA AGTTGTGGAG TGTAAGTTGT       6404
   CAAATGGAGA GGAAAATAAT ATG AAA AAA ATT TCA ATT TTA CAC TTT TCC          6454
                         Met Lys Lys Ile Ser Ile Leu His Phe Ser
                           1               5                  10
   CAA GTA TCA GGC GGG GGA GTT GAA AAG TAC ATA AAA TTA TTT TTA AAG        6502
   Gln Val Ser Gly Gly Gly Val Glu Lys Tyr Ile Lys Leu Phe Leu Lys
                    15                  20                  25
   TAT TCT GAT GTG ACA AAA TTT AAT AAT TAT TTA GTT GCA CCT AAT CTT        6550
   Tyr Ser Asp Val Thr Lys Phe Asn Asn Tyr Leu Val Ala Pro Asn Leu
                30                  35                  40
   GAA AAT TAT GAC GAA TTT AAT GGA TAT TTA AAG ATG TCT GTC AAT TTT        6598
   Glu Asn Tyr Asp Glu Phe Asn Gly Tyr Leu Lys Met Ser Val Asn Phe
            45                  50                  55
   AAT ATG GAA CAA ACT TTT TCT CCG CTA AAA ATA TTC AAA AAT GTC TTT        6646
   Asn Met Glu Gln Thr Phe Ser Pro Leu Lys Ile Phe Lys Asn Val Phe
        60                  65                  70
   TTT ATT CGT AGT GTA CTC AAA AAA ATA AAC CCA GAT ATA GTA TAC CTA        6694
   Phe Ile Arg Ser Val Leu Lys Lys Ile Asn Pro Asp Ile Val Tyr Leu
    75                  80                  85                  90
   CAT AGT ACA TTT GCA GGT GTC GTA GGT CGT ATT GCT TCA ATA GGT TTG      6742
   His Ser Thr Phe Ala Gly Val Val Gly Arg Ile Ala Ser Ile Gly Leu
                    95                  100                 105
   CCA ACA AAA GTA GTA TAC AAT CCT CAC GGA TGG TCC TTC AAA ATG GAC      6790
   Pro Thr Lys Val Val Tyr Asn Pro His Gly Trp Ser Phe Lys Met Asp
               110                 115                 120
   AAC AGC TAT TTG AAA AAG CTT ATT TTT AAA TTA ATC GAA TTT TCT TTA      6838
   Asn Ser Tyr Leu Lys Lys Leu Ile Phe Lys Leu Ile Glu Phe Ser Leu
           125                 130                 135
   TCT TTT TTA ACT GAT AAG TTT ATT TTA ATT TCG GAA TCT GAG TAT ATT      6886
   Ser Phe Leu Thr Asp Lys Phe Ile Leu Ile Ser Glu Ser Glu Tyr Ile
       140                 145                 150
   TTG GCT AAC CAT ATT TCA TTT AAT AAA AGC AAG TTT TCA CTA ATT AAT      6934
   Leu Ala Asn His Ile Ser Phe Asn Lys Ser Lys Phe Ser Leu Ile Asn
   155                 160                 165                 170
   AAT GGT GTT GAA GTG ATT ACA GGG GAT TCA AGA AAT GAG ATA GAA GAG      6982
   Asn Gly Val Glu Val Ile Thr Gly Asp Ser Arg Asn Glu Ile Glu Glu
                   175                 180                 185
   ATA TTT CCA AAT GAG GAT TTT ATA ATT GGC ATG GTT GGC AGA CTA AGC      7030
   Ile Phe Pro Asn Glu Asp Phe Ile Ile Gly Met Val Gly Arg Leu Ser
               190                 195                 200
   CCA CCC AAA GAG TTT TTC TTT TTT ATT GAT TTT GCA AAA AAA ATA TTA      7078
   Pro Pro Lys Glu Phe Phe Phe Phe Ile Asp Phe Ala Lys Lys Ile Leu
           205                 210                 215
   CAA ATT CGA AAC GAT ACC AAT TTT ATT ATC GTG GGT GAT GGA GAG TTA      7126
   Gln Ile Arg Asn Asp Thr Asn Phe Ile Ile Val Gly Asp Gly Glu Leu
       220                 225                 230
   CGA AGT GAA ATA GAA AGA ATG ATA CTA GAT AAT GGG TTA GGA GAT AAA      7174
   Arg Ser Glu Ile Glu Arg Met Ile Leu Asp Asn Gly Leu Gly Asp Lys
   235                 240                 245                 250
   ATC TAT ATT ACT GGG TGG GTT GAT AAT CCG AGA AAC TAT ATA GAG AAG      7222
   Ile Tyr Ile Thr Gly Trp Val Asp Asn Pro Arg Asn Tyr Ile Glu Lys
                   255                 260                 265
   TTT GAT CAA GCT ATT CTG TTT TCT AGA TGG GAG GGT CTT AGC CTA ACG      7270
   Phe Asp Gln Ala Ile Leu Phe Ser Arg Trp Glu Gly Leu Ser Leu Thr
               270                 275                 280
   ATT GCG GAA TAT ATG TCT CAG AAG AAA ACA ATT TTA GCA ACA AAT ATT      7318
   Ile Ala Glu Tyr Met Ser Gln Lys Lys Thr Ile Leu Ala Thr Asn Ile
           285                 290                 295
   GGT GGC ATT AAT GAT TTA ATC ACT GAT GGT GAA ACA GGA ATG CTG ATT      7366
   Gly Gly Ile Asn Asp Leu Ile Thr Asp Gly Glu Thr Gly Met Leu Ile
       300                 305                 310
   GAA GTT GGA GAC TTG AAT TCA GCA GTA TCT AAA TCT TTC GAG CTA AGA      7414
   Glu Val Gly Asp Leu Asn Ser Ala Val Ser Lys Ser Phe Glu Leu Arg
   315                 320                 325                 330
   AAT AAT AAA GAG GTT TCG AAT CAA TTA GCG AAT AAC GCT TAT AAT AAA      7462
   Asn Asn Lys Glu Val Ser Asn Gln Leu Ala Asn Asn Ala Tyr Asn Lys
                   335                 340                 345
   GTT GTT GAA CAG TTT TCG ATT GAA AAA CAG ATG GCT GAG ATA GAA AGT      7510
   Val Val Glu Gln Phe Ser Ile Glu Lys Gln Met Ala Glu Ile Glu Ser
               350                 355                 360
   TTA TTT ATA GAG ATG TGT AAC AAT GAG AAA TAGAGACTTA AAGAAAATAC        7560
   Leu Phe Ile Glu Met Cys Asn Asn Glu Lys
           365                 370
   AGGTTATTTG ATTGACTTAG AGTGGTCAAA ATCTGTCGAA GAAAAATTAT ATAAGGCAGC    7620
   GAAAATGGAT GCACAAATGA TTAAAGCAGA TTTTAGGCAA AGGTTTGCGA TTGATCAGAT    7680
   GTTAAAGCAA ATTAAAACAA TTTATTTAGC TTGAATGAAG AAAGAGGAGG CATAA ATG     7738
                                                                      Met
                                                                        1
   CTG ATT TTG AAA TTA AAA TTT CAT CTT AAA TCG TTA TTC CTT AAA TGG         7786
   Leu Ile Leu Lys Leu Lys Phe His Leu Lys Ser Leu Phe Leu Lys Trp
                 5                  10                  15
   ATT TAT CGA TTA CTT TAT CTA AAA AAG TTT CAG TTT GGT GCA CGC TTG         7834
   Ile Tyr Arg Leu Leu Tyr Leu Lys Lys Phe Gln Phe Gly Ala Arg Leu
            20                  25                  30
   ACG TTT CGA GAT GGG TTT CAT TTG TTA ATT GAA AAA TCT GGG AAA GTT         7882
   Thr Phe Arg Asp Gly Phe His Leu Leu Ile Glu Lys Ser Gly Lys Val
        35                  40                  45
   ATC ATC GGG AAT CAT GTT TTT TTT AAT AAC TTT TGT TCA ATT AAT GCC         7930
   Ile Ile Gly Asn His Val Phe Phe Asn Asn Phe Cys Ser Ile Asn Ala
    50                  55                  60                  65
   ATG TTA TCA GTA ACG ATT GGT GAT GAC TGT ATT TTT GGT GAA AAC GTT         7978
   Met Leu Ser Val Thr Ile Gly Asp Asp Cys Ile Phe Gly Glu Asn Val
                    70                  75                  80
   AAA ATT TAT GAT CAC AAT CAT TGT TAT CAA AAT AAA AGT CAA CCT ATT         8026
   Lys Ile Tyr Asp His Asn His Cys Tyr Gln Asn Lys Ser Gln Pro Ile
                85                  90                  95
   TCA AAA CAA GGT TTT TCA ACT GCT GCT ATC CAG ATT GGT CGT AAC TGT         8074
   Ser Lys Gln Gly Phe Ser Thr Ala Ala Ile Gln Ile Gly Arg Asn Cys
           100                 105                 110
   TGG ATA GGT AGT CAA GTG ACG ATT TTA AAA GGT GTA ACC ATA GGT GAT         8122
   Trp Ile Gly Ser Gln Val Thr Ile Leu Lys Gly Val Thr Ile Gly Asp
       115                 120                 125
   AAT AGT ATC ATT GGT GCT GGT GTG GTA GTT TAT CAA GAT GTG CCA GAA         8170
   Asn Ser Ile Ile Gly Ala Gly Val Val Val Tyr Gln Asp Val Pro Glu
   130                 135                 140                 145
   AAT TCG ATT GTT TTA TCT AAT GGA GAA ATT AGA AAG CGT GGC                 8212
   Asn Ser Ile Val Leu Ser Asn Gly Glu Ile Arg Lys Arg Gly
                   150                 155
   TAATTAAA ATG TAT CTT AAA AGT CTA ATC TCT ATT GTT ATT CCA GTA TAT        8262
            Met Tyr Leu Lys Ser Leu Ile Ser Ile Val Ile Pro Val Tyr
              1               5                  10
   AAT GTA GAG AAA TAT TTA GAA AAA TGT TTG CAA TCT GTT CAA AAT CAG         8310
   Asn Val Glu Lys Tyr Leu Glu Lys Cys Leu Gln Ser Val Gln Asn Gln
    15                  20                  25                  30
   ACT TAC AAT AAT TTT GAA GTG ATT TTA GTG AAT GAT GGC TCA ACC GAT         8358
   Thr Tyr Asn Asn Phe Glu Val Ile Leu Val Asn Asp Gly Ser Thr Asp
                    35                  40                  45
   TCA TCA CTT TCA ATA TGC GAA AAA TTT GTT AAT CAG GAT AAA AGA TTT         8406
   Ser Ser Leu Ser Ile Cys Glu Lys Phe Val Asn Gln Asp Lys Arg Phe
                50                  55                  60
   TCT GTT TTT TCT AAA GAA AAT GGT GGT ATG TCA TCT GCA CGA AAT TTT         8454
   Ser Val Phe Ser Lys Glu Asn Gly Gly Met Ser Ser Ala Arg Asn Phe
            65                  70                  75
   GGA ATT AAA AAG GCT AAA GGA TCG TTT ATC ACA TTT GTA GAT AGT GAT         8502
   Gly Ile Lys Lys Ala Lys Gly Ser Phe Ile Thr Phe Val Asp Ser Asp
        80                  85                  90
   GAC TAC ATA GTA AAA GAT TAT CTT TCT CAT TTG GTA GCT GGG ATA AAA         8550
   Asp Tyr Ile Val Lys Asp Tyr Leu Ser His Leu Val Ala Gly Ile Lys
    95                 100                 105                 110
   AGT GAG ACC TCT ATA GTT TGT TCA AAG TTT TTT CTT GTA GAT GAA AAA         8598
   Ser Glu Thr Ser Ile Val Cys Ser Lys Phe Phe Leu Val Asp Glu Lys
                   115                 120                 125
   GGA AGT TTA TTG ACT AAA AAA GAG GCA CCT AAA AAG AAA TCA GAA GTC         8646
   Gly Ser Leu Leu Thr Lys Lys Glu Ala Pro Lys Lys Lys Ser Glu Val
                    130                          135                          140
   GTT TCA ATT GAG GAA AGT ATT AAA ATT CTT CTG TTG CAA CAA AAT GGC        8694
   Val Ser Ile Glu Glu Ser Ile Lys Ile Leu Leu Leu Gln Gln Asn Gly
           145                     150                     155
   TAT GAT CTC GCT GTC TGG GGA AAA TTA TAC CCC GTT TCT TTC TTT GAA        8742
   Tyr Asp Leu Ala Val Trp Gly Lys Leu Tyr Pro Val Ser Phe Phe Glu
       160                     165                     170
   ACA ATT TCT TTC CCA GAA GGA AAA CTT TAC GAA GAT ATG GGA ACA ACT        8790
   Thr Ile Ser Phe Pro Glu Gly Lys Leu Tyr Glu Asp Met Gly Thr Thr
   175                     180                     185                     190
   TAC AAA TTA CTA AAA TTG GCA AGT GAA GTG GTC TTC TTG GAT GCG TAT        8838
   Tyr Lys Leu Leu Lys Leu Ala Ser Glu Val Val Phe Leu Asp Ala Tyr
                   195                     200                     205
   GAT TAT GCC TAC GTA CAG CGA CCT AAT AGT ATC ATG AAT AGT TCT TTT        8886
   Asp Tyr Ala Tyr Val Gln Arg Pro Asn Ser Ile Met Asn Ser Ser Phe
               210                     215                     220
   AAT TTG AAA AAG TTG GAT ATA ATA GAA ATG GTT CAT GAA ATG GAA AAC        8934
   Asn Leu Lys Lys Leu Asp Ile Ile Glu Met Val His Glu Met Glu Asn
           225                     230                     235
   GAT ATA TTA GCA CAG TTT CCA AAT TTA GCA TTA TAT GTT AAG AAT CGA        8982
   Asp Ile Leu Ala Gln Phe Pro Asn Leu Ala Leu Tyr Val Lys Asn Arg
       240                     245                     250
   GCA TTT GCC GCG GAA GTG AAA ATC TTT TTA GAG ATT CCA AAA GAA AAA        9030
   Ala Phe Ala Ala Glu Val Lys Ile Phe Leu Glu Ile Pro Lys Glu Lys
   255                     260                     265                     270
   GAA TTT GAG CAA GCG CAA AAG CAA CTT TGG CAT GAT ATC AAA AAG AAT        9078
   Glu Phe Glu Gln Ala Gln Lys Gln Leu Trp His Asp Ile Lys Lys Asn
                   275                     280                     285
   AGA AAA GCA CCA TTT ATG ACA AAA GGT GCT AGA TTG AAG AAT AGG CTC        9126
   Arg Lys Ala Pro Phe Met Thr Lys Gly Ala Arg Leu Lys Asn Arg Leu
               290                     295                     300
   GGA GCT AGT CTG TCG TTT TTA GGT AAA TCT TTA TTT TTG ACT ATT GGG        9174
   Gly Ala Ser Leu Ser Phe Leu Gly Lys Ser Leu Phe Leu Thr Ile Gly
           305                     310                     315
   AAG CAG TTA GTA GAT AGA TAATGATATT GAAAGCGATA CGATACAATC               9222
   Lys Gln Leu Val Asp Arg
       320
   GTAAACTTCT TTTGGTGTTG ACTAGGAGTT AGCTTGAAAT TTGAATATAA AGGAAGCAAC      9282
   AC ATG GTA ATT TAT TTT TTA CTT TTC CCG ATG ATC GCA ATG ATT TAT         9329
      Met Val Ile Tyr Phe Leu Leu Phe Pro Met Ile Ala Met Ile Tyr
        1               5                  10                  15
   TTA ATG ACA TTG CTC TTA CGG CAA AAA GCA CAA ATC CAA AAA ACG ATT        9377
   Leu Met Thr Leu Leu Leu Arg Gln Lys Ala Gln Ile Gln Lys Thr Ile
                   20                  25                  30
   TTT TGT GTT CTT ACG TTT GGT ACA CTA GGC TTT ATT TCA GCA AGT CGT        9425
   Phe Cys Val Leu Thr Phe Gly Thr Leu Gly Phe Ile Ser Ala Ser Arg
               35                  40                  45
   GCA TCA AGT GTT GGG ACG GAC GTT ACT TTA TAC GAA AAT ATT TTT AAA        9473
   Ala Ser Ser Val Gly Thr Asp Val Thr Leu Tyr Glu Asn Ile Phe Lys
           50                  55                  60
   TCT ATA AAT TAC GGG ATA AGT GCT GAG AAT AAT TGG GGA TAC GTC ATC        9521
   Ser Ile Asn Tyr Gly Ile Ser Ala Glu Asn Asn Trp Gly Tyr Val Ile
       65                  70                  75
   TAT AAC AAG CTG ATT GGT AGT GTA TTT GGC TAT ACA GGA CAT GAA ATC        9569
   Tyr Asn Lys Leu Ile Gly Ser Val Phe Gly Tyr Thr Gly His Glu Ile
   80                  85                  90                  95
   ACG GCC GCT AAT TCA GTT TTG ATT ACA ATA CTT ATT GGT ATT TTT ATT 9617
   Thr Ala Ala Asn Ser Val Leu Ile Thr Ile Leu Ile Gly Ile Phe Ile
   100 105 110
   TGG AAA GTA GCG GAA CAT TAT TTT GTT GCG ACG TTT TTA TAC ATT AGC 9665
   Trp Lys Val Ala Glu His Tyr Phe Val Ala Thr Phe Leu Tyr Ile Ser
   115 120 125
   TTG TTT TAT TAT GCT ACA AGT TTT AAT ATT TCA AGA CAA TTT ATT GCC 9713
   Leu Phe Tyr Tyr Ala Thr Ser Phe Asn Ile Ser Arg Gln Phe Ile Ala
   130 135 140
   ATG GGG CTT GTA TTG GTA GCA ATT TCT TTT GCT TTA GAT AAA AAG GTT 9761
   Met Gly Leu Val Leu Val Ala Ile Ser Phe Ala Leu Asp Lys Lys Val
   145 150 155
   ATG CCT TGG TTT ATC TTG ACA GTT TTG GCT ACC TTA TTT CAT GCG ACA 9809
   Met Pro Trp Phe Ile Leu Thr Val Leu Ala Thr Leu Phe His Ala Thr
   160 165 170 175
   GCA ATC GTT GCT TTT CCT GTC TAT TGG CTT ACA AAA GTA CAT TGG GAT 9857
   Ala Ile Val Ala Phe Pro Val Tyr Trp Leu Thr Lys Val His Trp Asp
   180 185 190
   GTG AAA AAG ACA TTA AGT ATT TTT CCA ATC ACG ATT TTT GCA AGT TTT 9905
   Val Lys Lys Thr Leu Ser Ile Phe Pro Ile Thr Ile Phe Ala Ser Phe
   195 200 205
   ATT TTT GAT GCT ATT TTA AAC ATT TTT GTA CGT TTT TTC CCA CAT TAT 9953
   Ile Phe Asp Ala Ile Leu Asn Ile Phe Val Arg Phe Phe Pro His Tyr
   210 215 220
   GAG ATG TAT ATC ACT GGA ACA CAA TTT AAT ATT TCA GAT CAG GGG CAG 10001
   Glu Met Tyr Ile Thr Gly Thr Gln Phe Asn Ile Ser Asp Gln Gly Gln
   225 230 235
   GGA CGT GTG GTT TTG GTC AAA ATA TTT ATC TTG CTC ATT TTG TTT ACT 10049
   Gly Arg Val Val Leu Val Lys Ile Phe Ile Leu Leu Ile Leu Phe Thr
   240 245 250 255
   TTA TTC TTG TTT TAT AAA AAA AGC TAT GCT TTG ATT TCT GAA TGT CAT 10097
   Leu Phe Leu Phe Tyr Lys Lys Ser Tyr Ala Leu Ile Ser Glu Cys His
   260 265 270
   CAA AGT TTG ATA GCT TTG ACA ACC GTT GGA TTA AGT ATC GGT ATT GTA 10145
   Gln Ser Leu Ile Ala Leu Thr Thr Val Gly Leu Ser Ile Gly Ile Val
   275 280 285
   TTT TAT AAT AAT ATT TTA CTC AAT AGA ATA GAA ATG TTT TAT TCA ATT 10193
   Phe Tyr Asn Asn Ile Leu Leu Asn Arg Ile Glu Met Phe Tyr Ser Ile
   290 295 300
   TTA AGC ATC GTA TTT ATT CCA ATT GCT ATA GAT TAC ATT AGT TTG AAA 10241
   Leu Ser Ile Val Phe Ile Pro Ile Ala Ile Asp Tyr Ile Ser Leu Lys
   305 310 315
   TTT AAA CAA AAA GAT GCT GTG CGA CTA ATG CTG ACG ATA GGT ATT TTG 10289
   Phe Lys Gln Lys Asp Ala Val Arg Leu Met Leu Thr Ile Gly Ile Leu
   320 325 330 335
   TTA ATT ACA CTT GTG CCT TAC TAT ATA CAG GTT AGC GGT AAT TAT TCA 10337
   Leu Ile Thr Leu Val Pro Tyr Tyr Ile Gln Val Ser Gly Asn Tyr Ser
   340 345 350
   GGA ATA TTG CCT TAT GTT ATT CAA CAA TAAAAAATAA AGTTTAGAGA 10384
   Gly Ile Leu Pro Tyr Val Ile Gln Gln
   355 360
   GGAAATA ATG GAG GAT AGA AAG AAA CAA GTA ATT TTG ATA CTA TCC CAC 10433
   Met Glu Asp Arg Lys Lys Gln Val Ile Leu Ile Leu Ser His
   1 5 10
   AGA AAT ACT CTC GCT CTA AAA TCA ACA ATA GAG CTT TTG GAT TCT CAA 10481
   Arg Asn Thr Leu Ala Leu Lys Ser Thr Ile Glu Leu Leu Asp Ser Gln
   15 20 25 30
   TAC TTT GAT TTC TTT CTT CAT ATA GAT AAA AAA AGT AGA ATT CAA GAT    10529
   Tyr Phe Asp Phe Phe Leu His Ile Asp Lys Lys Ser Arg Ile Gln Asp
                    35                  40                  45
   TTT TTT TAT TTA AAA AAA ATT ACA AAA TTC TCC ACT ATT CAT TTT TCA    10577
   Phe Phe Tyr Leu Lys Lys Ile Thr Lys Phe Ser Thr Ile His Phe Ser
                50                  55                  60
   GAA AGA AAA AAT GTA CAT TGG GGA GGT TTT TCT ATG GTA GAA GCA ATG    10625
   Glu Arg Lys Asn Val His Trp Gly Gly Phe Ser Met Val Glu Ala Met
            65                  70                  75
   TTT GCG CTA TTA GAA TGT GCA CGT GAT ACA GGA GAA TAT TCT TAT TTT    10673
   Phe Ala Leu Leu Glu Cys Ala Arg Asp Thr Gly Glu Tyr Ser Tyr Phe
        80                  85                  90
   CAT TTT TTA TCT GGA GAT GAT ATG CCA ATC AAA GAT AAT GAA ATA GTA    10721
   His Phe Leu Ser Gly Asp Asp Met Pro Ile Lys Asp Asn Glu Ile Val
    95                 100                 105                 110
   TTT AAT TTT TTT GAA AAT AGT TAT CCT AAA AAT TTT ATT GAT ATT CTA    10769
   Phe Asn Phe Phe Glu Asn Ser Tyr Pro Lys Asn Phe Ile Asp Ile Leu
                   115                 120                 125
   GAT TTT GAA AAT GTC AAT AAA AAT TCA TAT TTC TAC GAA CCC CCT GAG    10817
   Asp Phe Glu Asn Val Asn Lys Asn Ser Tyr Phe Tyr Glu Pro Pro Glu
               130                 135                 140
   ATG ATA GAG GAG AGA GTG AAG TAC TAC TAT CCT CAT ATG GAT ATT CTA    10865
   Met Ile Glu Glu Arg Val Lys Tyr Tyr Tyr Pro His Met Asp Ile Leu
           145                 150                 155
   AAC AGA AAA GGA ACA AAT TTC ATA GGG AAA AAA CTA ATT TAT CTA CAA    10913
   Asn Arg Lys Gly Thr Asn Phe Ile Gly Lys Lys Leu Ile Tyr Leu Gln
       160                 165                 170
   AAA TTG TTG AAA GTT AAT CGC TTG AAA AAT AGA GAG ATA GAA ATT TTC    10961
   Lys Leu Leu Lys Val Asn Arg Leu Lys Asn Arg Glu Ile Glu Ile Phe
   175                 180                 185                 190
   AAG GGT CAT CAA TGG TGT AGT TTG ACA AAT CAA TTT GTA GAT ATT TTA    11009
   Lys Gly His Gln Trp Cys Ser Leu Thr Asn Gln Phe Val Asp Ile Leu
                   195                 200                 205
   TTG GAT AAA GAG GAA AGA AGA GTA GGT AAG TCT TAT TTT TCA TCT AGT    11057
   Leu Asp Lys Glu Glu Arg Arg Val Gly Lys Ser Tyr Phe Ser Ser Ser
               210                 215                 220
   TTA ATA CCA GAT GAA TGT TAT TTT CAA ACG TTT GCT ATG ATA AAA AAA    11105
   Leu Ile Pro Asp Glu Cys Tyr Phe Gln Thr Phe Ala Met Ile Lys Lys
           225                 230                 235
   GTT GAA ATT TAT CAA CAG AAA AAT ATG TCA GCA CGC TTA ATT GAT TGG    11153
   Val Glu Ile Tyr Gln Gln Lys Asn Met Ser Ala Arg Leu Ile Asp Trp
       240                 245                 250
   ACA AGA GGG AAA CCA TAT ATT TGG CGA CAG GAT GAT TTT TTT GAA ATT    11201
   Thr Arg Gly Lys Pro Tyr Ile Trp Arg Gln Asp Asp Phe Phe Glu Ile
   255                 260                 265                 270
   ATG AAT GAT AAA GAT TCA ATG TTT TCT AGG AAG TTT GAT GAA AAT GTA    11249
   Met Asn Asp Lys Asp Ser Met Phe Ser Arg Lys Phe Asp Glu Asn Val
                   275                 280                 285
   GAT CGT AAA ATA ATT GAA GAA ATT TAT ATA AAA ATA AGA GGA AGA AGT    11297
   Asp Arg Lys Ile Ile Glu Glu Ile Tyr Ile Lys Ile Arg Gly Arg Ser
               290                 295                 300
   ACT GAT GAA GCA AAT AAA ATC AAA GAT AAG AGA TTT ACA AAA            11339
   Thr Asp Glu Ala Asn Lys Ile Lys Asp Lys Arg Phe Thr Lys
           305                 310                 315
   TAATTTTACC TATGTTTTTG GAAAGAAAAC TTTTCTTGGA AGGGGAGAAG CGATTATCAT  11399
   AGATGAACCT GAGCATGGAA ATTTGGGAGA TCAAGCAATT GCTTTTGCAG AAAATCAATT  11459
   TTTAGTAAAT CATGTATCAG TACGAGATGT AGAACATCTT ATAGAAAGCA AAACTATTTC  11519
   AGAAATAAAA TCTATAAAAA AAAATATTGG AAAAAAAGAA TTAGTTTTTT TTCATGGGGG 11579
   AGGAAATTTC GGGACACTTT ATCTAAAGTA TGAGCGCATT AGAAGATTGG CAGTATCAAA 11639
   GCTTCCCTTT AATAAAATGA TTCTATTTCC TCAGTCAATT TCATTTGAAG ATAGTAGGTT 11699
   TGGTCAGAAG CAGCTGAATA AAAGTAAAAA AATATACAGT CAAAATACAA ATTTTATTTT 11759
   GACTGCAAGA GAACCAAAAT CTTATGGTTT AATGAAGAAA TGTTTTCCAT ATAACAAAGT 11819
   AATCTTGACA CCGGATATCG TGCTCTCATT TAAATTTGAA GTCACCATTT CTGATACGCA 11879
   TATTGGGAAA GAAAAGGATA GTGTTATAAC TTATGAAAAT CGTCAACACT ATCTTGAGAT 11939
   AAAGTGGGAT GAAATTGCGC AGCATGAGGT CGCCTTAACT GATAGATTAC ATGGTATGAT 11999
   TTTTTCATAT ATCACAGGCA CACCATGTGT TGTTTTGGCT AATAATAATC ATAAAATTGA 12059
   AGGAACATAC AAACATTGGT TGAATGAAGT CAACTATATT CGTTTTATTG AAAATCCGAC 12119
   TGTTGAAAAT ATTTTAGATG CAATCAATGA CTTAAAGCAA ATCGAACCTC ACTATATTGA 12179
   TTTATCTGAT AAATTTCAAC CACTAATTGA TGCGATAAAA GGGTAAAGGT TTA ATG 12235
   Met
   1
   AAT AAA TAT AAA AAA CTA CTA TCC AAC TCT CTT GTT TTC ACG ATA GGA 12283
   Asn Lys Tyr Lys Lys Leu Leu Ser Asn Ser Leu Val Phe Thr Ile Gly
   5 10 15
   AAC TTA GGC AGC AAA CTG TTA GTC TTT TTA CTC GTA CCG CTC TAC ACC 12331
   Asn Leu Gly Ser Lys Leu Leu Val Phe Leu Leu Val Pro Leu Tyr Thr
   20 25 30
   TAT GCG ATG ACA CCG CAA GAG TAT GGT ATG GCA GAC TTA TAT CAA ACA 12379
   Tyr Ala Met Thr Pro Gln Glu Tyr Gly Met Ala Asp Leu Tyr Gln Thr
   35 40 45
   ACA GCA AAT CTA CTT TTG CCA TTA ATT ACA ATG AAT GTA TTT GAT GCA 12427
   Thr Ala Asn Leu Leu Leu Pro Leu Ile Thr Met Asn Val Phe Asp Ala
   50 55 60 65
   ACT TTA CGT TTT GCT ATG GAA AAG TCA ATG ACA AAA GAG AGT GTG TTA 12475
   Thr Leu Arg Phe Ala Met Glu Lys Ser Met Thr Lys Glu Ser Val Leu
   70 75 80
   ACA AAT TCT CTT GTG GTT TGG TGT TTT AGC GCG GTG TTC ACT TGT TTG 12523
   Thr Asn Ser Leu Val Val Trp Cys Phe Ser Ala Val Phe Thr Cys Leu
   85 90 95
   GGC GCT TGT ATT ATC TAT GCG TTG AAC TTG AGT AAT AAA TGG TAT TTA 12571
   Gly Ala Cys Ile Ile Tyr Ala Leu Asn Leu Ser Asn Lys Trp Tyr Leu
   100 105 110
   GCT TTA CTT TTA ACC TTC AAC TTA TTT CAA GGT GGA CAA AGT ATA TTA 12619
   Ala Leu Leu Leu Thr Phe Asn Leu Phe Gln Gly Gly Gln Ser Ile Leu
   115 120 125
   AGC CAG TAT GCT AGA GGT ATA GGA AAG TCG AAA ATA TTT GCA GCT GGC 12667
   Ser Gln Tyr Ala Arg Gly Ile Gly Lys Ser Lys Ile Phe Ala Ala Gly
   130 135 140 145
   GGA GTT ATT TTA ACC TTT TTG ACA GGC GCT TTA AAT ATT CTT TTT TTG 12715
   Gly Val Ile Leu Thr Phe Leu Thr Gly Ala Leu Asn Ile Leu Phe Leu
   150 155 160
   GTA TAT TTA CCG CTT GGG ATT ACG GGC TAT TTA ATG TCC CTG GTT TTA 12763
   Val Tyr Leu Pro Leu Gly Ile Thr Gly Tyr Leu Met Ser Leu Val Leu
   165 170 175
   GCG AAT GTA GGT ACG ATT CTA TTT TTT GCT GGC ACA CTT TCC ATT TGG 12811
   Ala Asn Val Gly Thr Ile Leu Phe Phe Ala Gly Thr Leu Ser Ile Trp
   180 185 190
   AAG GAA ATT AGT TTT AAA ATA ATT GAT AAA AAA CTG ATT TGG CAA ATG 12859
   Lys Glu Ile Ser Phe Lys Ile Ile Asp Lys Lys Leu Ile Trp Gln Met
           195                 200                 205
   CTC TAT TAT GCC TTA CCT TTG ATT CCT AGT TCC ATC CTG TGG TGG TTA      12907
   Leu Tyr Tyr Ala Leu Pro Leu Ile Pro Ser Ser Ile Leu Trp Trp Leu
   210                 215                 220                 225
   CTG AAT GCT TCT AGT CGC TAT TTC GTT TTA TTC TTT TTA GGA GCA GGT      12955
   Leu Asn Ala Ser Ser Arg Tyr Phe Val Leu Phe Phe Leu Gly Ala Gly
                   230                 235                 240
   GCT AAT GGT CTT TTG GCG GTC GCT ACC AAA ATT CCA AGT ATT ATT TCC      13003
   Ala Asn Gly Leu Leu Ala Val Ala Thr Lys Ile Pro Ser Ile Ile Ser
               245                 250                 255
   ATT TTT AAT ACG ATT TTT ACA CAG GCG TGG CAA ATT TCA GCC ATA GAA      13051
   Ile Phe Asn Thr Ile Phe Thr Gln Ala Trp Gln Ile Ser Ala Ile Glu
           260                 265                 270
   GAA TAT GAT TCT CAT CAA AAA TCA AAA TAT TAT TCG GAT GTT TTT CAC      13099
   Glu Tyr Asp Ser His Gln Lys Ser Lys Tyr Tyr Ser Asp Val Phe His
       275                 280                 285
   TAC TTA GCA ACT TTT CTA TTG TTA GGG ACA TCA GCT TTT ATG ATT GTG      13147
   Tyr Leu Ala Thr Phe Leu Leu Leu Gly Thr Ser Ala Phe Met Ile Val
   290                 295                 300                 305
   CTT AAA CCA ATT GTC GAA AAA GTC GTT TCA AGT GAC TAT GCA AGT TCA      13195
   Leu Lys Pro Ile Val Glu Lys Val Val Ser Ser Asp Tyr Ala Ser Ser
                   310                 315                 320
   TGG CAA TAT GTT CCT TTC TTT ATG TTG TCG ATG CTA TTT TCC TCG TTT      13243
   Trp Gln Tyr Val Pro Phe Phe Met Leu Ser Met Leu Phe Ser Ser Phe
               325                 330                 335
   TCT GAT TTT TTT GGG ACT AAT TAT ATT GCG GCT AAA CAA ACA AAA GGC      13291
   Ser Asp Phe Phe Gly Thr Asn Tyr Ile Ala Ala Lys Gln Thr Lys Gly
           340                 345                 350
   GTA TTT ATG ACA TCT ATC TAT GGT ACC ATT GTT TGT GTC TTA CTC CAA      13339
   Val Phe Met Thr Ser Ile Tyr Gly Thr Ile Val Cys Val Leu Leu Gln
       355                 360                 365
   GTG GTG CTG CTA CCC ATC ATC GGC TTG GAT GGC GCA GGT TTA TCA GCC      13387
   Val Val Leu Leu Pro Ile Ile Gly Leu Asp Gly Ala Gly Leu Ser Ala
   370                 375                 380                 385
   ATG CTT GGA TTT TTA ACA ACG TTT TTA TTG CGT GTC AAA GAT ACG CAA      13435
   Met Leu Gly Phe Leu Thr Thr Phe Leu Leu Arg Val Lys Asp Thr Gln
                   390                 395                 400
   AAA TTT GTG GTG ATT CAG ATT AAG TGG CGG ATT TTT ATC AGT AAT TTA      13483
   Lys Phe Val Val Ile Gln Ile Lys Trp Arg Ile Phe Ile Ser Asn Leu
               405                 410                 415
   TTG ATC GTT TTG GCA CAA ATT TTA TGT TTG TTT TAT CTA CCG AGT GAA      13531
   Leu Ile Val Leu Ala Gln Ile Leu Cys Leu Phe Tyr Leu Pro Ser Glu
           420                 425                 430
   TTT TTG TAT TTT GGT CTT GCC CTA TTA TTT TGT GGC ATG TTA GTG GTT      13579
   Phe Leu Tyr Phe Gly Leu Ala Leu Leu Phe Cys Gly Met Leu Val Val
       435                 440                 445
   AAT CAG CGT ACA ATT TTA TAC ATT ATC ATG GCG CTA AAA ATA AAA AAT      13627
   Asn Gln Arg Thr Ile Leu Tyr Ile Ile Met Ala Leu Lys Ile Lys Asn
   450                 455                 460                 465
   AAG ACA TTT GGA ATG AAA TCC TCA TAAAAATAGA CAGGAGGTGT ATCTCGAATG      13681
   Lys Thr Phe Gly Met Lys Ser Ser
                   470
   GTATCGAGAT ATATCTCCTG TCTATTTTTA TGATACTTTT GTGTTAGCTC AACTCAACCG     13741
   CCTTTTAATC TCCCAACAAC AATAATACCC AATCAAACAA CCCAAAAAAT TCAAGATAAT     13801
   ATCACTAATG GCAAATGTGC CCAAATAAAA GATAAATTGA ATGGTTTCAA TTACTAAAAG     13861
   AGTGACCAAA CTGACAATGA CAAACTGTTT GAAATCAGTA TTGATACAGT AAAGGCCACC     13921
   TAAAGGAATG AAGTAGATAA TATTTAGCAC AGCCTCTTGA ATCGTTCTGG GATCCGCTTT   13981
   TATAAAGTCA AAAGGATTCA GTGACATCGC CTGAAAATCC GTTATTTTAG TAAAAAGTAC   14041
   CATGAATAAC AGTAATAAAT ACACACTGAA AGCAAGATAG AGATAAATAA CTGAAAAATA   14101
   TTTGAGGTGA TACTGGATAC CAAACAACCA GATAATCAGC GTTAATAAGA GTATTAAAGT   14161
   CAATGTGGTA TAGTCAAAGT GGTTAATCAA CTTAGCCAGG CTTTGATAGC GAGTGAGAAC   14221
   GGGCATAATC AGCCAAGTAA TCGTCGCATA ACTCAGGATA AATGTGATCA ATAAACTGCT   14281
   GAGGTAGATC ATATATTTTC GCAACTGTTT CTAACTCCTT TTCTTGATGA GATTAACCCT   14341
   ATTTTAACAT ATTTTAAAAC TGTCATGTTT TTATGAATTT AAAATAAATG TTAAAGAAAA   14401
   TAAAAATTCA CCAGTTGGTT CTGTTGCAAA GTTTTCCAAA AAATCTATTT TAGTGTAAAA   14461
   TTGAGAAAAA AGACAGAGAG GACAGAGTAA TGAATTATTT TAAAGGCAAA CAATTCAAAA   14521
   AAGACGTCAT TATTGTCTCT GTTGGTTACT ACCTGCGTTA CAATCTAAGC TATCGTTAAG   14581
   TTCAGGAATT GTTATATGAT C                                             14602
(2) INFORMATIONS POUR LA SEQ ID NO: 2:
   (i) CARACTERISTIQUES DE LA SEQUENCE:
      (A) LONGUEUR: 484 acides aminés
      (B) TYPE: acide aminé
      (D) CONFIGURATION: linéaire
   (ii) TYPE DE MOLECULE: protéine
   (xi) DESCRIPTION DE LA SEQUENCE: SEQ ID NO: 2:
   Met Ser Ser Arg Thr Asn Arg Lys Gln Lys His Thr Ser Asn Gly Ser
    1               5                  10                  15
   Trp Gly Met Val Asn Val Gly Leu Thr Ile Leu Tyr Ala Ile Leu Ala
               20                  25                  30
   Leu Val Leu Leu Phe Thr Met Phe Asn Tyr Asn Phe Leu Ser Phe Arg
           35                  40                  45
   Phe Leu Asn Ile Ile Ile Thr Ile Gly Leu Leu Val Val Leu Ala Ile
       50                  55                  60
   Ser Ile Phe Leu Gln Lys Thr Lys Lys Leu Pro Leu Val Thr Thr Val
   65                  70                  75                  80
   Val Leu Val Ile Phe Ser Leu Val Ser Leu Val Gly Ile Phe Gly Phe
                   85                  90                  95
   Lys Gln Met Ile Asp Ile Thr Asn Arg Met Asn Gln Thr Ala Ala Phe
               100                 105                 110
   Ser Glu Val Glu Met Ser Ile Val Val Pro Lys Glu Ser Asp Ile Lys
           115                 120                 125
   Asp Val Ser Gln Leu Thr Ser Val Gln Ala Pro Thr Lys Val Asp Lys
       130                 135                 140
   Asn Asn Ile Glu Ile Leu Met Ser Ala Leu Lys Lys Asp Lys Lys Val
   145                 150                 155                 160
   Asp Val Lys Val Asp Asp Val Ala Ser Tyr Gln Glu Ala Tyr Asp Asn
                   165                 170                 175
   Leu Lys Ser Gly Lys Ser Lys Ala Met Val Leu Ser Gly Ser Tyr Ala
               180                 185                 190
   Ser Leu Leu Glu Ser Val Asp Ser Asn Tyr Ala Ser Asn Leu Lys Thr
           195                 200                 205
   Ile Tyr Thr Tyr Lys Ile Lys Lys Lys Asn Ser Asn Ser Ala Asn Gln
       210                 215                 220
   Val Asp Ser Arg Val Phe Asn Ile Tyr Ile Ser Gly Ile Asp Thr Tyr
   225                 230                 235                 240
   Gly Pro Ile Ser Thr Val Ser Arg Ser Asp Val Asn Ile Ile Met Thr
                   245                 250                 255
   Val Asn Met Asn Thr His Lys Ile Leu Leu Thr Thr Thr Pro Arg Asp
               260                 265                 270
   Ala Tyr Val Lys Ile Pro Gly Gly Gly Ala Asp Gln Tyr Asp Lys Leu
           275                 280                 285
   Thr His Ala Gly Ile Tyr Gly Val Glu Thr Ser Glu Gln Thr Leu Glu
       290                 295                 300
   Asp Leu Tyr Gly Ile Lys Leu Asp Tyr Tyr Ala Arg Ile Asn Phe Thr
   305                 310                 315                 320
   Ser Phe Leu Lys Leu Ile Asp Gln Leu Gly Gly Val Thr Val His Asn
                   325                 330                 335
   Asp Gln Ala Phe Thr Gln Glu Lys Phe Asp Phe Pro Val Gly Asp Ile
               340                 345                 350
   Gln Met Asn Ser Glu Gln Ala Leu Gly Phe Val Arg Glu Arg Tyr Asn
           355                 360                 365
   Leu Asp Gly Gly Asp Asn Asp Arg Gly Lys Asn Gln Glu Lys Val Ile
       370                 375                 380
   Ser Ala Ile Leu Asn Lys Leu Ala Ser Leu Lys Ser Val Ser Asn Phe
   385                 390                 395                 400
   Thr Ser Ile Val Asn Asn Leu Gln Asp Ser Val Gln Thr Asn Met Ser
                   405                 410                 415
   Leu Asn Thr Ile Asn Ala Leu Ala Asn Thr Gln Leu Glu Ser Gly Ser
               420                 425                 430
   Lys Phe Thr Val Thr Ser Gln Ala Val Thr Gly Thr Gly Ser Thr Gly
           435                 440                 445
   Gln Leu Ile Ser Tyr Ala Met Pro Asn Ser Ser Leu Tyr Met Met Lys
       450                 455                 460
   Leu Asp Asn Ser Ser Val Glu Ser Ala Ser Gln Ala Ile Lys Lys Leu
   465                 470                 475                 480
   Met Glu Glu Lys
(2) INFORMATIONS POUR LA SEQ ID NO: 3:
   (i) CARACTERISTIQUES DE LA SEQUENCE:
      (A) LONGUEUR: 243 acides aminés
      (B) TYPE: acide aminé
      (D) CONFIGURATION: linéaire
   (ii) TYPE DE MOLECULE: protéine
   (xi) DESCRIPTION DE LA SEQUENCE: SEQ ID NO: 3:
   Val Ile Asp Val His Ser His Ile Val Phe Asp Val Asp Asp Gly Pro
    1              5                   10                  15
   Glu Thr Leu Glu Glu Ser Leu Asp Leu Ile Gly Glu Ser Tyr Ala Gln
               20                  25                  30
   Gly Val Arg Lys Ile Val Ser Thr Ser His Arg Arg Lys Gly Met Phe
           35                  40                  45
   Glu Thr Pro Glu Asp Lys Ile Phe Ala Asn Phe Lys Lys Val Lys Ala
       50                  55                  60
   Glu Ala Glu Ala Leu Tyr Pro Asp Leu Thr Ile Tyr Tyr Gly Gly Glu
   65                  70                  75                  80
   Leu Tyr Tyr Thr Ser Asp Ile Val Glu Lys Leu Glu Lys Asn Leu Ile
                   85                  90                  95
   Pro Arg Met His Asn Thr Gln Phe Ala Leu Ile Glu Phe Ser Ala Arg
               100                 105                 110
   Thr Ser Trp Lys Glu Ile His Ser Gly Leu Ser Asn Val Leu Arg Ala
           115                 120                 125
   Gly Val Thr Pro Ile Val Ala His Ile Glu Arg Tyr Asp Ala Leu Glu
       130                 135                 140
   Glu Asn Ala Asp Arg Val Arg Glu Ile Ile Asn Met Gly Cys Tyr Thr
   145                 150                 155                 160
   Gln Val Asn Ser Ser His Val Leu Lys Pro Lys Leu Phe Gly Asp Lys
                   165                 170                 175
   Asp Lys Val Arg Lys Lys Arg Val Arg Phe Phe Leu Glu Lys Asn Leu
               180                 185                 190
   Val His Met Val Ala Ser Asp Met His Asn Leu Gly Pro Arg Pro Pro
           195                 200                 205
   Phe Met Lys Asp Ala Tyr Glu Ile Val Lys Lys Asn Tyr Gly Ser Lys
       210                 215                 220
   Arg Ala Lys Asn Leu Phe Ile Glu Asn Pro Lys Thr Leu Leu Glu Asn
   225                 230                 235                 240
   Gln Tyr Leu
(2) INFORMATIONS POUR LA SEQ ID NO: 4:
   (i) CARACTERISTIQUES DE LA SEQUENCE:
      (A) LONGUEUR: 231 acides aminés
      (B) TYPE: acide aminé
      (D) CONFIGURATION: linéaire
   (ii) TYPE DE MOLECULE: protéine
   (xi) DESCRIPTION DE LA SEQUENCE: SEQ ID NO: 4:
(2) INFORMATIONS POUR LA SEQ ID NO: 5:
   (i) CARACTERISTIQUES DE LA SEQUENCE:
      (A) LONGUEUR: 249 acides aminés
      (B) TYPE: acide aminé
      (D) CONFIGURATION: linéaire
   (ii) TYPE DE MOLECULE: protéine
   (xi) DESCRIPTION DE LA SEQUENCE: SEQ ID NO: 5:
   Met Pro Leu Leu Lys Leu Val Lys Ser Lys Val Asp Phe Ala Lys Lys
    1               5                  10                  15
   Thr Glu Glu Tyr Tyr Asn Ala Ile Arg Thr Asn Ile Gln Phe Ser Gly
               20                  25                  30
   Ala Gln Met Lys Val Ile Ala Ile Ser Ser Val Glu Ala Gly Glu Gly
           35                  40                  45
   Lys Ser Met Ile Ser Val Asn Leu Ala Ile Ser Phe Ala Ser Val Gly
       50                  55                  60
   Leu Arg Thr Leu Leu Ile Asp Ala Glu Thr Arg Asn Ser Val Leu Ser
   65                  70                  75                  80
   Gly Thr Phe Lys Ser Asn Glu Pro Tyr Lys Gly Leu Ser Asn Phe Leu
                   85                  90                  95
   Ser Gly Asn Ala Asp Leu Asn Glu Thr Ile Cys Gln Thr Asp Ile Ser
               100                 105                 110
   Gly Leu Asp Val Ile Ala Ser Gly Pro Val Pro Pro Asn Pro Thr Ser
           115                 120                 125
   Leu Leu Gln Asn Asp Asn Phe Arg His Leu Met Glu Val Ala Arg Ser
       130                 135                 140
   Cys Tyr Asp Tyr Val Ile Ile Asp Thr Pro Pro Val Gly Leu Val Ile
   145                 150                 155                 160
   Asp Ala Val Ile Ile Ala His Gln Ala Asp Ala Ser Leu Leu Val Thr
                   165                 170                 175
   Glu Ala Gly Lys Ile Lys Arg Arg Phe Val Thr Lys Ala Val Glu Gln
               180                 185                 190
   Leu Val Glu Ser Gly Ser Gln Phe Leu Gly Val Val Leu Asn Lys Val
           195                 200                 205
   Asp Met Thr Val Asp Lys Tyr Gly Phe Tyr Gly Ser Tyr Gly Ser Tyr
       210                 215                 220
   Gly Glu Tyr Gly Lys Lys Ser Asp Gln Lys Glu Gly His Ser Arg Ala
   225                 230                 235                 240
   His Arg Arg Arg Lys Val Gly Trp Asn
                   245
(2) INFORMATIONS POUR LA SEQ ID NO: 6:
   (i) CARACTERISTIQUES DE LA SEQUENCE:
      (A) LONGUEUR: 227 acides aminés
      (B) TYPE: acide aminé
      (D) CONFIGURATION: linéaire
   (ii) TYPE DE MOLECULE: protéine
   (xi) DESCRIPTION DE LA SEQUENCE: SEQ ID NO: 6:
   Met Ser Gln Ala Lys Glu Glu Ile Ser Asp Val Met Thr Tyr Ser Glu
    1               5                  10                  15
   Leu Thr Ser His Lys Pro Lys Ile Ile Tyr Ser Leu Ile Lys Arg Ile
               20                  25                  30
   Gly Asp Ile Leu Val Ser Ser Ile Gly Leu Ile Ile Leu Ile Pro Leu
           35                  40                  45
   Phe Leu Ile Val Ala Leu Ile Met Lys Cys Ser Glu Pro Thr Ala Pro
       50                  55                  60
   Ile Phe Phe Ser His Ile Arg Asn Gly Lys Asn Gly Lys Lys Phe Lys
   65                  70                  75                  80
   Met Tyr Lys Phe Arg Thr Met Cys Gln Asp Ala Glu Ser Ile Leu Met
                   85                  90                  95
   Lys Asp Thr Glu Leu Phe Ala Lys Phe Lys Ala Asn Gly Tyr Lys Leu
               100                 105                 110
   Glu Thr His Glu Asp Pro Arg Ile Thr Lys Ile Gly Gly Ile Leu Arg
           115                 120                 125
   Lys Thr Ser Ile Asp Glu Leu Pro Gln Leu Ile Asn Val Phe Leu Gly
       130                 135                 140
   Gln Met Ser Leu Val Gly Pro Arg Pro Leu Pro Asp Arg Glu Ile Ile
   145                 150                 155                 160
   Glu Tyr Gly Asp Asn Gln Glu Lys Phe Leu Ser Val Lys Pro Gly Met
                   165                 170                 175
   Thr Gly Trp Trp Gln Val Ser Gly Arg Ser Thr Ile Gly Tyr Pro Glu
               180                 185                 190
   Arg Cys His Leu Glu Leu Tyr Tyr Val Glu Lys Cys Cys Phe Thr Phe
           195                 200                 205
   Asp Val Leu Ile Leu Leu Lys Thr Ile Gly Ile Val Leu Lys Arg Val
       210                 215                 220
   Gly Ala Arg
   225
(2) INFORMATIONS POUR LA SEQ ID NO: 7:
   (i) CARACTERISTIQUES DE LA SEQUENCE:
      (A) LONGUEUR: 319 acides aminés
      (B) TYPE: acide aminé
      (D) CONFIGURATION: linéaire
   (ii) TYPE DE MOLECULE: protéine
   (xi) DESCRIPTION DE LA SEQUENCE: SEQ ID NO: 7:
   Met Asn Glu Gln Val Thr Phe Ile Leu Cys Asp Phe Leu Val Arg Glu
    1               5                  10                  15
   Ile Lys Pro Lys Tyr Asp Leu Leu Ala Tyr Gln Phe Ile Ser Lys Lys
               20                  25                  30
   Ile Lys Glu Ile Lys Pro Asp Ile Val His Cys His Ser Ser Lys Ala
           35                  40                  45
   Gly Val Ile Gly Arg Leu Ala Ala Lys Arg Arg Gly Val Lys Lys Ile
       50                  55                  60
   Phe Tyr Thr Pro His Ala Tyr Ser Phe Leu Ala Pro Glu Phe Ser Gly
   65                  70                  75                  80
   Lys Lys Lys Phe Leu Phe Val Gln Ile Glu Lys Phe Leu Ser Arg Phe
                   85                  90                  95
   Ala Thr Thr Lys Ile Phe Cys Val Ser Ile Ala Glu Met Gln Ala Ala
               100                 105                 110
   Leu Glu Val Asn Leu Asp Lys Thr Asp Lys Phe Gln Val Ile Tyr Asn
           115                 120                 125
   Gly Leu Pro Glu Ile Asp Leu Pro Ser Lys Glu Thr Ile Arg Ala Gln
       130                 135                 140
   Leu Gly Leu Glu Lys Ala Ala Val Val Ile Gly Asn Asn Ala Lys Met
   145                 150                 155                 160
   Ser Glu Gln Lys Asn Pro Met Phe Phe Met Glu Ile Ala Arg Lys Met
                   165                 170                 175
   Ile Arg Gln Asn Ala Asn Trp His Phe Val Trp Val Gly Asp Gly Gln
               180                 185                 190
   Leu Met Pro Leu Phe Gln Ser Phe Ile Lys Gln Asn Gly Leu Glu Gly
           195                 200                 205
   Asn Ile His Leu Leu Gly Glu Arg Pro Asp Ser Glu Ile Val Val Thr
       210                 215                 220
   Ala Tyr Asp Ile Phe Leu Thr Thr Ser Gln Tyr Glu Gly Leu Pro Tyr
   225                 230                 235                 240
   Ala Pro Ile Glu Ala Met Arg Ala Gly Val Pro Ile Leu Ala Thr Lys
                   245                 250                 255
   Val Val Gly Asn Ser Glu Leu Val Ile Glu Gly Lys Asn Gly Tyr Leu
               260                 265                 270
   Ile Asp Leu Glu Trp Ser Lys Ser Val Glu Glu Lys Leu Tyr Lys Ala
           275                 280                 285
   Ala Lys Ile Asp Ala Gln Met Ile Lys Ala Asp Phe Arg Gln Arg Phe
       290                 295                 300
   Ala Ile Asp Gln Ile Leu Lys Gln Ile Glu Thr Ile Tyr Leu Ala
   305                 310                 315
(2) INFORMATIONS POUR LA SEQ ID NO: 8:
   (i) CARACTERISTIQUES DE LA SEQUENCE:
      (A) LONGUEUR: 372 acides aminés
      (B) TYPE: acide aminé
      (D) CONFIGURATION: linéaire
   (ii) TYPE DE MOLECULE: protéine
   (xi) DESCRIPTION DE LA SEQUENCE: SEQ ID NO: 8:
   Met Lys Lys Ile Ser Ile Leu His Phe Ser Gln Val Ser Gly Gly Gly
    1               5                   10                  15
   Val Glu Lys Tyr Ile Lys Leu Phe Leu Lys Tyr Ser Asp Val Thr Lys
               20                  25                  30
   Phe Asn Asn Tyr Leu Val Ala Pro Asn Leu Glu Asn Tyr Asp Glu Phe
           35                  40                  45
   Asn Gly Tyr Leu Lys Met Ser Val Asn Phe Asn Met Glu Gln Thr Phe
       50                  55                  60
   Ser Pro Leu Lys Ile Phe Lys Asn Val Phe Phe Ile Arg Ser Val Leu
   65                  70                  75                  80
   Lys Lys Ile Asn Pro Asp Ile Val Tyr Leu His Ser Thr Phe Ala Gly
                   85                  90                  95
   Val Val Gly Arg Ile Ala Ser Ile Gly Leu Pro Thr Lys Val Val Tyr
               100                 105                 110
   Asn Pro His Gly Trp Ser Phe Lys Met Asp Asn Ser Tyr Leu Lys Lys
           115                 120                 125
   Leu Ile Phe Lys Leu Ile Glu Phe Ser Leu Ser Phe Leu Thr Asp Lys
       130                 135                 140
   Phe Ile Leu Ile Ser Glu Ser Glu Tyr Ile Leu Ala Asn His Ile Ser
   145                 150                 155                 160
   Phe Asn Lys Ser Lys Phe Ser Leu Ile Asn Asn Gly Val Glu Val Ile
                   165                 170                 175
   Thr Gly Asp Ser Arg Asn Glu Ile Glu Glu Ile Phe Pro Asn Glu Asp
               180                 185                 190
   Phe Ile Ile Gly Met Val Gly Arg Leu Ser Pro Pro Lys Glu Phe Phe
           195                 200                 205
   Phe Phe Ile Asp Phe Ala Lys Lys Ile Leu Gln Ile Arg Asn Asp Thr
       210                 215                 220
   Asn Phe Ile Ile Val Gly Asp Gly Glu Leu Arg Ser Glu Ile Glu Arg
   225                 230                 235                 240
   Met Ile Leu Asp Asn Gly Leu Gly Asp Lys Ile Tyr Ile Thr Gly Trp
                   245                 250                 255
   Val Asp Asn Pro Arg Asn Tyr Ile Glu Lys Phe Asp Gln Ala Ile Leu
               260                 265                 270
   Phe Ser Arg Trp Glu Gly Leu Ser Leu Thr Ile Ala Glu Tyr Met Ser
           275                 280                 285
   Gln Lys Lys Thr Ile Leu Ala Thr Asn Ile Gly Gly Ile Asn Asp Leu
       290                 295                 300
   Ile Thr Asp Gly Glu Thr Gly Met Leu Ile Glu Val Gly Asp Leu Asn
   305                 310                 315                 320
   Ser Ala Val Ser Lys Ser Phe Glu Leu Arg Asn Asn Lys Glu Val Ser
                   325                 330                 335
   Asn Gln Leu Ala Asn Asn Ala Tyr Asn Lys Val Val Glu Gln Phe Ser
               340                 345                 350
   Ile Glu Lys Gln Met Ala Glu Ile Glu Ser Leu Phe Ile Glu Met Cys
           355                 360                 365
   Asn Asn Glu Lys
       370
(2) INFORMATIONS POUR LA SEQ ID NO: 9:
   (i) CARACTERISTIQUES DE LA SEQUENCE:
      (A) LONGUEUR: 159 acides aminés
      (B) TYPE: acide aminé
      (D) CONFIGURATION: linéaire
   (ii) TYPE DE MOLECULE: protéine
   (xi) DESCRIPTION DE LA SEQUENCE: SEQ ID NO: 9:
   Met Leu Ile Leu Lys Leu Lys Phe His Leu Lys Ser Leu Phe Leu Lys
   1               5                   10                  15
   Trp Ile Tyr Arg Leu Leu Tyr Leu Lys Lys Phe Gln Phe Gly Ala Arg
               20                  25                  30
   Leu Thr Phe Arg Asp Gly Phe His Leu Leu Ile Glu Lys Ser Gly Lys
           35                  40                  45
   Val Ile Ile Gly Asn His Val Phe Phe Asn Asn Phe Cys Ser Ile Asn
       50                  55                  60
   Ala Met Leu Ser Val Thr Ile Gly Asp Asp Cys Ile Phe Gly Glu Asn
   65                  70                  75                  80
   Val Lys Ile Tyr Asp His Asn His Cys Tyr Gln Asn Lys Ser Gln Pro
                   85                  90                  95
   Ile Ser Lys Gln Gly Phe Ser Thr Ala Ala Ile Gln Ile Gly Arg Asn
               100                 105                 110
   Cys Trp Ile Gly Ser Gln Val Thr Ile Leu Lys Gly Val Thr Ile Gly
           115                 120                 125
   Asp Asn Ser Ile Ile Gly Ala Gly Val Val Val Tyr Gln Asp Val Pro
       130                 135                 140
   Glu Asn Ser Ile Val Leu Ser Asn Gly Glu Ile Arg Lys Arg Gly
   145                 150                 155
(2) INFORMATIONS POUR LA SEQ ID NO: 10:
   (i) CARACTERISTIQUES DE LA SEQUENCE:
      (A) LONGUEUR: 324 acides aminés
      (B) TYPE: acide aminé
      (D) CONFIGURATION: linéaire
   (ii) TYPE DE MOLECULE: protéine
   (xi) DESCRIPTION DE LA SEQUENCE: SEQ ID NO: 10:
   Met Tyr Leu Lys Ser Leu Ile Ser Ile Val Ile Pro Val Tyr Asn Val
    1               5                  10                  15
   Glu Lys Tyr Leu Glu Lys Cys Leu Gln Ser Val Gln Asn Gln Thr Tyr
               20                  25                  30
   Asn Asn Phe Glu Val Ile Leu Val Asn Asp Gly Ser Thr Asp Ser Ser
           35                  40                  45
   Leu Ser Ile Cys Glu Lys Phe Val Asn Gln Asp Lys Arg Phe Ser Val
       50                  55                  60
   Phe Ser Lys Glu Asn Gly Gly Met Ser Ser Ala Arg Asn Phe Gly Ile
   65                  70                  75                  80
   Lys Lys Ala Lys Gly Ser Phe Ile Thr Phe Val Asp Ser Asp Asp Tyr
                   85                  90                  95
   Ile Val Lys Asp Tyr Leu Ser His Leu Val Ala Gly Ile Lys Ser Glu
               100                 105                 110
   Thr Ser Ile Val Cys Ser Lys Phe Phe Leu Val Asp Glu Lys Gly Ser
           115                 120                 125
   Leu Leu Thr Lys Lys Glu Ala Pro Lys Lys Lys Ser Glu Val Val Ser
       130                 135                 140
   Ile Glu Glu Ser Ile Lys Ile Leu Leu Leu Gln Gln Asn Gly Tyr Asp
   145                 150                 155                 160
   Leu Ala Val Trp Gly Lys Leu Tyr Pro Val Ser Phe Phe Glu Thr Ile
                   165                 170                 175
   Ser Phe Pro Glu Gly Lys Leu Tyr Glu Asp Met Gly Thr Thr Tyr Lys
               180                 185                 190
   Leu Leu Lys Leu Ala Ser Glu Val Val Phe Leu Asp Ala Tyr Asp Tyr
           195                 200                 205
   Ala Tyr Val Gln Arg Pro Asn Ser Ile Met Asn Ser Ser Phe Asn Leu
       210                 215                 220
   Lys Lys Leu Asp Ile Ile Glu Met Val His Glu Met Glu Asn Asp Ile
   225                 230                 235                 240
   Leu Ala Gln Phe Pro Asn Leu Ala Leu Tyr Val Lys Asn Arg Ala Phe
                   245                 250                 255
   Ala Ala Glu Val Lys Ile Phe Leu Glu Ile Pro Lys Glu Lys Glu Phe
               260                 265                 270
   Glu Gln Ala Gln Lys Gln Leu Trp His Asp Ile Lys Lys Asn Arg Lys
           275                 280                 285
   Ala Pro Phe Met Thr Lys Gly Ala Arg Leu Lys Asn Arg Leu Gly Ala
       290                 295                 300
   Ser Leu Ser Phe Leu Gly Lys Ser Leu Phe Leu Thr Ile Gly Lys Gln
   305                 310                 315                 320
   Leu Val Asp Arg
(2) INFORMATIONS POUR LA SEQ ID NO: 11:
   (i) CARACTERISTIQUES DE LA SEQUENCE:
      (A) LONGUEUR: 360 acides aminés
      (B) TYPE: acide aminé
      (D) CONFIGURATION: linéaire
   (ii) TYPE DE MOLECULE: protéine
   (xi) DESCRIPTION DE LA SEQUENCE: SEQ ID NO: 11:
   Met Val Ile Tyr Phe Leu Leu Phe Pro Met Ile Ala Met Ile Tyr Leu
    1               5                  10                  15
   Met Thr Leu Leu Leu Arg Gln Lys Ala Gln Ile Gln Lys Thr Ile Phe
               20                  25                  30
   Cys Val Leu Thr Phe Gly Thr Leu Gly Phe Ile Ser Ala Ser Arg Ala
           35                  40                  45
   Ser Ser Val Gly Thr Asp Val Thr Leu Tyr Glu Asn Ile Phe Lys Ser
       50                  55                  60
   Ile Asn Tyr Gly Ile Ser Ala Glu Asn Asn Trp Gly Tyr Val Ile Tyr
    65                  70                  75                  80
   Asn Lys Leu Ile Gly Ser Val Phe Gly Tyr Thr Gly His Glu Ile Thr
                   85                  90                  95
   Ala Ala Asn Ser Val Leu Ile Thr Ile Leu Ile Gly Ile Phe Ile Trp
               100                 105                 110
   Lys Val Ala Glu His Tyr Phe Val Ala Thr Phe Leu Tyr Ile Ser Leu
           115                 120                 125
   Phe Tyr Tyr Ala Thr Ser Phe Asn Ile Ser Arg Gln Phe Ile Ala Met
       130                 135                 140
   Gly Leu Val Leu Val Ala Ile Ser Phe Ala Leu Asp Lys Lys Val Met
   145                 150                 155                 160
   Pro Trp Phe Ile Leu Thr Val Leu Ala Thr Leu Phe His Ala Thr Ala
                   165                 170                 175
   Ile Val Ala Phe Pro Val Tyr Trp Leu Thr Lys Val His Trp Asp Val
               180                 185                 190
   Lys Lys Thr Leu Ser Ile Phe Pro Ile Thr Ile Phe Ala Ser Phe Ile
           195                 200                 205
   Phe Asp Ala Ile Leu Asn Ile Phe Val Arg Phe Phe Pro His Tyr Glu
       210                 215                 220
   Met Tyr Ile Thr Gly Thr Gln Phe Asn Ile Ser Asp Gln Gly Gln Gly
   225                 230                 235                 240
   Arg Val Val Leu Val Lys Ile Phe Ile Leu Leu Ile Leu Phe Thr Leu
                   245                 250                 255
   Phe Leu Phe Tyr Lys Lys Ser Tyr Ala Leu Ile Ser Glu Cys His Gln
               260                 265                 270
   Ser Leu Ile Ala Leu Thr Thr Val Gly Leu Ser Ile Gly Ile Val Phe
           275                 280                 285
   Tyr Asn Asn Ile Leu Leu Asn Arg Ile Glu Met Phe Tyr Ser Ile Leu
       290                 295                 300
   Ser Ile Val Phe Ile Pro Ile Ala Ile Asp Tyr Ile Ser Leu Lys Phe
   305                 310                 315                 320
   Lys Gln Lys Asp Ala Val Arg Leu Met Leu Thr Ile Gly Ile Leu Leu
                   325                 330                 335
   Ile Thr Leu Val Pro Tyr Tyr Ile Gln Val Ser Gly Asn Tyr Ser Gly
               340                 345                 350
   Ile Leu Pro Tyr Val Ile Gln Gln
           355                 360
(2) INFORMATIONS POUR LA SEQ ID NO: 12:
   (i) CARACTERISTIQUES DE LA SEQUENCE:
      (A) LONGUEUR: 316 acides aminés
      (B) TYPE: acide aminé
      (D) CONFIGURATION: linéaire
   (ii) TYPE DE MOLECULE: protéine
   (xi) DESCRIPTION DE LA SEQUENCE: SEQ ID NO: 12:
   Met Glu Asp Arg Lys Lys Gln Val Ile Leu Ile Leu Ser His Arg Asn
   1 5 10 15
   Thr Leu Ala Leu Lys Ser Thr Ile Glu Leu Leu Asp Ser Gln Tyr Phe
   20 25 30
   Asp Phe Phe Leu His Ile Asp Lys Lys Ser Arg Ile Gln Asp Phe Phe
   35 40 45
   Tyr Leu Lys Lys Ile Thr Lys Phe Ser Thr Ile His Phe Ser Glu Arg
   50 55 60
   Lys Asn Val His Trp Gly Gly Phe Ser Met Val Glu Ala Met Phe Ala
   65 70 75 80
   Leu Leu Glu Cys Ala Arg Asp Thr Gly Glu Tyr Ser Tyr Phe His Phe
   85 90 95
   Leu Ser Gly Asp Asp Met Pro Ile Lys Asp Asn Glu Ile Val Phe Asn
   100 105 110
   Phe Phe Glu Asn Ser Tyr Pro Lys Asn Phe Ile Asp Ile Leu Asp Phe
   115 120 125
   Glu Asn Val Asn Lys Asn Ser Tyr Phe Tyr Glu Pro Pro Glu Met Ile
   130 135 140
   Glu Glu Arg Val Lys Tyr Tyr Tyr Pro His Met Asp Ile Leu Asn Arg
   145 150 155 160
   Lys Gly Thr Asn Phe Ile Gly Lys Lys Leu Ile Tyr Leu Gln Lys Leu
   165 170 175
   Leu Lys Val Asn Arg Leu Lys Asn Arg Glu Ile Glu Ile Phe Lys Gly
   180 185 190
   His Gln Trp Cys Ser Leu Thr Asn Gln Phe Val Asp Ile Leu Leu Asp
   195 200 205
   Lys Glu Glu Arg Arg Val Gly Lys Ser Tyr Phe Ser Ser Ser Leu Ile
   210 215 220
   Pro Asp Glu Cys Tyr Phe Gln Thr Phe Ala Met Ile Lys Lys Val Glu
   225 230 235 240
   Ile Tyr Gln Gln Lys Asn Met Ser Ala Arg Leu Ile Asp Trp Thr Arg
   245 250 255
   Gly Lys Pro Tyr Ile Trp Arg Gln Asp Asp Phe Phe Glu Ile Met Asn
   260 265 270
   Asp Lys Asp Ser Met Phe Ser Arg Lys Phe Asp Glu Asn Val Asp Arg
   275 280 285
   Lys Ile Ile Glu Glu Ile Tyr Ile Lys Ile Arg Gly Arg Ser Thr Asp
   290 295 300
   Glu Ala Asn Lys Ile Lys Asp Lys Arg Phe Thr Lys
   305 310 315
(2) INFORMATIONS POUR LA SEQ ID NO: 13:
   (i) CARACTERISTIQUES DE LA SEQUENCE:
      (A) LONGUEUR: 473 acides aminés
      (B) TYPE: acide aminé
      (D) CONFIGURATION: linéaire
   (ii) TYPE DE MOLECULE: protéine
   (xi) DESCRIPTION DE LA SEQUENCE: SEQ ID NO: 13:
   Met Asn Lys Tyr Lys Lys Leu Leu Ser Asn Ser Leu Val Phe Thr Ile
    1               5                  10                  15
   Gly Asn Leu Gly Ser Lys Leu Leu Val Phe Leu Leu Val Pro Leu Tyr
               20                  25                  30
   Thr Tyr Ala Met Thr Pro Gln Glu Tyr Gly Met Ala Asp Leu Tyr Gln
           35                  40                  45
   Thr Thr Ala Asn Leu Leu Leu Pro Leu Ile Thr Met Asn Val Phe Asp
       50                  55                  60
   Ala Thr Leu Arg Phe Ala Met Glu Lys Ser Met Thr Lys Glu Ser Val
   65                  70                  75                  80
   Leu Thr Asn Ser Leu Val Val Trp Cys Phe Ser Ala Val Phe Thr Cys
                   85                  90                  95
   Leu Gly Ala Cys Ile Ile Tyr Ala Leu Asn Leu Ser Asn Lys Trp Tyr
               100                 105                 110
   Leu Ala Leu Leu Leu Thr Phe Asn Leu Phe Gln Gly Gly Gln Ser Ile
           115                 120                 125
   Leu Ser Gln Tyr Ala Arg Gly Ile Gly Lys Ser Lys Ile Phe Ala Ala
       130                 135                 140
   Gly Gly Val Ile Leu Thr Phe Leu Thr Gly Ala Leu Asn Ile Leu Phe
   145                 150                 155                 160
   Leu Val Tyr Leu Pro Leu Gly Ile Thr Gly Tyr Leu Met Ser Leu Val
                   165                 170                 175
   Leu Ala Asn Val Gly Thr Ile Leu Phe Phe Ala Gly Thr Leu Ser Ile
               180                 185                 190
   Trp Lys Glu Ile Ser Phe Lys Ile Ile Asp Lys Lys Leu Ile Trp Gln
           195                 200                 205
   Met Leu Tyr Tyr Ala Leu Pro Leu Ile Pro Ser Ser Ile Leu Trp Trp
       210                 215                 220
   Leu Leu Asn Ala Ser Ser Arg Tyr Phe Val Leu Phe Phe Leu Gly Ala
   225                 230                 235                 240
   Gly Ala Asn Gly Leu Leu Ala Val Ala Thr Lys Ile Pro Ser Ile Ile
                   245                 250                 255
   Ser Ile Phe Asn Thr Ile Phe Thr Gln Ala Trp Gln Ile Ser Ala Ile
               260                 265                 270
   Glu Glu Tyr Asp Ser His Gln Lys Ser Lys Tyr Tyr Ser Asp Val Phe
           275                 280                 285
   His Tyr Leu Ala Thr Phe Leu Leu Leu Gly Thr Ser Ala Phe Met Ile
       290                 295                 300
   Val Leu Lys Pro Ile Val Glu Lys Val Val Ser Ser Asp Tyr Ala Ser
   305                 310                 315                 320
   Ser Trp Gln Tyr Val Pro Phe Phe Met Leu Ser Met Leu Phe Ser Ser
                   325                 330                 335
   Phe Ser Asp Phe Phe Gly Thr Asn Tyr Ile Ala Ala Lys Gln Thr Lys
               340                 345                 350
   Gly Val Phe Met Thr Ser Ile Tyr Gly Thr Ile Val Cys Val Leu Leu
           355                 360                 365
   Gln Val Val Leu Leu Pro Ile Ile Gly Leu Asp Gly Ala Gly Leu Ser
       370                 375                 380
   Ala Met Leu Gly Phe Leu Thr Thr Phe Leu Leu Arg Val Lys Asp Thr
   385                 390                 395                 400
   Gln Lys Phe Val Val Ile Gln Ile Lys Trp Arg Ile Phe Ile Ser Asn
                   405                 410                 415
   Leu Leu Ile Val Leu Ala Gln Ile Leu Cys Leu Phe Tyr Leu Pro Ser
               420                 425                 430
   Glu Phe Leu Tyr Phe Gly Leu Ala Leu Leu Phe Cys Gly Met Leu Val
           435                 440                 445
   Val Asn Gln Arg Thr Ile Leu Tyr Ile Ile Met Ala Leu Lys Ile Lys
       450                 455                 460
   Asn Lys Thr Phe Gly Met Lys Ser Ser
   465                 470
(2) INFORMATIONS POUR LA SEQ ID NO: 14:
   (i) CARACTERISTIQUES DE LA SEQUENCE:
      (A) LONGUEUR: 307 acides aminés
      (B) TYPE: acide aminé
      (C) NOMBRE DE BRINS: simple
      (D) CONFIGURATION: linéaire
   (ii) TYPE DE MOLECULE: peptide
   (xi) DESCRIPTION DE LA SEQUENCE: SEQ ID NO: 14:
   Met Lys Gln Ile Lys Ser Lys Ile Arg Asp Leu Gln Asn Asn Phe Thr
   1               5                   10                  15
   Tyr Val Phe Gly Lys Lys Thr Phe Leu Gly Arg Gly Glu Ala Ile Ile
               20                  25                  30
   Ile Asp Glu Pro Glu His Gly Asn Leu Gly Asp Gln Ala Ile Ala Phe
           35                  40                  45
   Ala Glu Asn Gln Phe Leu Val Asn His Val Ser Val Arg Asp Val Glu
       50                  55                  60
   His Leu Ile Glu Ser Lys Thr Ile Ser Glu Ile Lys Ser Ile Lys Lys
   65                  70                  75                  80
   Asn Ile Gly Lys Lys Glu Leu Val Phe Phe His Gly Gly Gly Asn Phe
                   85                  90                  95
   Gly Thr Leu Tyr Leu Lys Tyr Glu Arg Ile Arg Arg Leu Ala Val Ser
               100                 105                 110
   Lys Leu Pro Phe Asn Lys Met Ile Leu Phe Pro Gln Ser Ile Ser Phe
           115                 120                 125
   Glu Asp Ser Arg Phe Gly Gln Lys Gln Leu Asn Lys Ser Lys Lys Ile
       130                 135                 140
   Tyr Ser Gln Asn Thr Asn Phe Ile Leu Thr Ala Arg Glu Pro Lys Ser
   145                 150                 155                 160
   Tyr Gly Leu Met Lys Lys Cys Phe Pro Tyr Asn Lys Val Ile Leu Thr
                   165                 170                 175
   Pro Asp Ile Val Leu Ser Phe Lys Phe Glu Val Thr Ile Ser Asp Thr
               180                 185                 190
   His Ile Gly Lys Glu Lys Asp Ser Val Ile Thr Tyr Glu Asn Arg Gln
           195                 200                 205
   His Tyr Leu Glu Ile Lys Trp Asp Glu Ile Ala Gln His Glu Val Ala
       210                 215                 220
   Leu Thr Asp Arg Leu His Gly Met Ile Phe Ser Tyr Ile Thr Gly Thr
   225                 230                 235                 240
   Pro Cys Val Val Leu Ala Asn Asn Asn His Lys Ile Glu Gly Thr Tyr
                   245                 250                 255
   Lys His Trp Leu Asn Glu Val Asn Tyr Ile Arg Phe Ile Glu Asn Pro
               260                 265                 270
   Thr Val Glu Asn Ile Leu Asp Ala Ile Asn Asp Leu Lys Gln Ile Glu
           275                 280                 285
   Pro His Tyr Ile Asp Leu Ser Asp Lys Phe Gln Pro Leu Ile Asp Ala
       290                 295                 300
   Ile Lys Gly
   305
(2) INFORMATIONS POUR LA SEQ ID NO: 15:
   (i) CARACTERISTIQUES DE LA SEQUENCE:
      (A) LONGUEUR: 32 paires de bases
      (B) TYPE: nucléotide
      (C) NOMBRE DE BRINS: simple
      (D) CONFIGURATION: linéaire
   (ii) TYPE DE MOLECULE: Autre acide nucl,ique
      (A) DESCRIPTION: /desc = "oligonuceotide"
   (xi) DESCRIPTION DE LA SEQUENCE: SEQ ID NO: 15:
   GTTGCGGCCG CGATAAAGTG TGATAAGTCC AG 32
(2) INFORMATIONS POUR LA SEQ ID NO: 16:
   (i) CARACTERISTIQUES DE LA SEQUENCE:
      (A) LONGUEUR: 30 paires de bases
      (B) TYPE: nucléotide
      (C) NOMBRE DE BRINS: simple
      (D) CONFIGURATION: linéaire
   (ii) TYPE DE MOLECULE: Autre acide nucléique
      (A) DESCRIPTION: /desc = "oligonucleotide"
   (xi) DESCRIPTION DE LA SEQUENCE: SEO ID NO: 16:
   ATAGCGGCCG CTTAGCTCAT GTTGATGCGG 30
(2) INFORMATIONS POUR LA SEQ ID NO: 17:
   (i) CARACTERISTIQUES DE LA SEQUENCE:
      (A) LONGUEUR: 31 paires de bases
      (B) TYPE: nucléotide
      (C) NOMBRE DE BRINS: simple
      (D) CONFIGURATION: linéaire
   (ii) TYPE DE MOLECULE: Autre acide nucl, ique
      (A) DESCRIPTION: /desc = "oligonucleotide"
   (xi) DESCRIPTION DE LA SEQUENCE: SEQ ID NO: 17:
   CCTGCGGCCG CGCTTCCTAA TTCTGTAATC G 31
(2) INFORMATIONS POUR LA SEQ ID NO: 18:
   (i) CARACTERISTIQUES DE LA SEQUENCE:
      (A) LONGUEUR: 31 paires de bases
      (B) TYPE: nucléotide
      (C) NOMBRE DE BRINS: simple
      (D) CONFIGURATION: linéaire
   (ii) TYPE DE MOLECULE: Autre acide nucl,ique
      (A) DESCRIPTION: /desc = "oligonucleotide"
   (xi) DESCRIPTION DE LA SEQUENCE: SEQ ID NO: 18:
   CTGGCGGCCG CTACTTCACG TTTCTTTGCA T 31
(2) INFORMATIONS POUR LA SEQ ID NO: 19:
   (i) CARACTERISTIQUES DE LA SEQUENCE:
      (A) LONGUEUR: 31 paires de bases
      (B) TYPE: nucléotide
      (C) NOMBRE DE BRINS: simple
      (D) CONFIGURATION: linéaire
   (ii) TYPE DE MOLECULE: Autre acide nucl,ique
      (A) DESCRIPTION: /desc = "oligonucleotide"
   (xi) DESCRIPTION DE LA SEQUENCE: SEQ ID NO: 19:
   TACGCGGCCG CACATAGAAT AAGGCTTTAC G                                    31

## Description

La présente invention se rapporte à l'utilisation de fragments d'ADN chromosomique de bactéries lactiques codant pour au moins une enzyme impliquée dans la biosynthèse d'exopolysaccharides, ainsi que des enzymes codées par ces fragments.

### Etat de la technique

Il est connu que les bactéries lactiques sont susceptibles de produire dans leur milieu de culture deux classes de polysaccharides, à savoir les homopolysaccharides comme les dextranes ou les levanes qui sont constitués par l'assemblage répété d'un seul sucre, et les hétéropolysaccharides appellés communément exopolysaccharides ou EPS (EPS est l'abréviation du terme "exopolysaccharide") constitués par l'assemblage de plusieurs sucres différents formant une unité répétitive (Cerning J., Bactéries lactiques, Vol I, de Rossart H et Luquet F. M., Lorica, 309-329, 1994).

Une bactérie lactique produisant un EPS peut conférer un caractère filant et/ou une texture lisse et crémeuse à un lait acidifié (Cerning *et al*., FEMS Microbiol., 87, 113-130, 19/90). Les EPS peuvent aussi présenter des activités biologiques particulièrement intéressantes pour la santé humaine ou animale, comme des activités anti-tumeurs ou probiotiques, par exemple (Oda M. *et al*., Agric. Biol. Chem., 47, 1623-1625, 1983; EP94870139.6)

Par ailleurs, l'industrie est confrontée à une instabilité génétique de la biosynthèse des EPS dans les bactéries lactiques. Ceci se traduit généralement au cours d'une fermentation par la perte de la production d'EPS par tout ou partie des bactéries lactiques (voir "Cerning J." ci-dessus). Les produits fermentés industriels sont ainsi sujets à des variations dans leur contenu en EPS, ce qui n'est pas toujours acceptable. Pour remédier à ces problèmes, l'industrie recours actuellement à l'isolation et la caractérisation périodique de ses bactéries de manière à séparer celles qui ont perdu leur caractère originel.

La biosynthèse d'EPS dans les bactéries lactiques mésophiles, c'est à dire les bactéries lactiques ayant une croissance optimale à 28-37°C, implique au moins une enzyme qui assure l'enchaînement des sucres. Aucun gène chromosomique ou plasmidique de bactéries lactiques mésophiles codant pour une telle enzyme n'a encore été identifié et séquencé, bien que l'on connaisse des plasmides impliqués dans la biosynthèse d'EPS.

WO 92/02142 révèle ainsi l'existence du plasmide pHV67 qui produit dans *Lactococcus lactis* subsp. *lactis* (mésophile) une substance capable d'augmenter la viscosité d'un lait fermenté. US5066588 décrit deux plasmides provenant d'une souche de *Streptococcus cremoris* (mésophile) capable de confèrer un caractère épaississant à un *Streptococcus lactis.* De même, Vescovo *et al.* ont mis en évidence un plasmide d'une souche *Lactobacillus casei* subsp. *casei* (mésophile) codant pour un phénotype Muc+, c'est à dire pour des fonctions liées à la production d'épaississants exocellulaires (Vescovo *et al*., Biotechnology Letters, Vol II, 709-712, 1989).

Enfin, Van den Berg *et al.* cherchent à isoler d'un *Lactobacillus sake* (mésophile) un groupe de gènes chromosomiques impliqués dans la biosynthèse d'un EPS (Van den Berg D.J.C. *et al*., First International Conference on Polysaccharide Engineering, Trondheim, Norway, June 6-8, 1994). Cependant aucun gène n'a encore été identifié et/ou séquencé.

D'un autre coté, la biosynthèse d'EPS dans les bactéries lactiques thermophiles, c'est à dire les bactéries lactiques ayant une croissance optimale à 37-45°C, n'est pas encore bien connue. On sait cependant qu'elle n'est pas associée à un plasmide. Vescovo *et al*. ont ainsi montré que le phénotype Muc+ de la souche *Lactobacillus delbrueckii* subsp *bulgaricus* 2o1 (thermophile) est lié à des fonctions chromosomiques (Vescoso *et al*., Biotechnology Letters, Vol II, 709-712, 1989).

Ainsi à ce jour, aucun gène ou groupe de gènes chromosomiques ou plasmidiques codant pour un EPS de bactéries lactiques mésophiles ou thermophiles n'a été identifié et/ou séquencé.

Il serait donc très intéressant d'avoir des moyens pour restaurer ou stabiliser la production originelle d'EPS dans les bactéries lactiques. De plus, il serait également intéressant d'avoir des moyens pour modifier la structure d'un EPS, et créer de ce fait de nouveaux EPS pouvant avoir des propriétés intéressantes.

La publication de Stingele et al. (Dev. Biol. Stand., Vol. 85, pp. 487-493 (1995)) décrit deux méthodes de "gene-tagging" servant à identifier les gènes impliqués dans la production d'exopolysaccharides. Ce document se réfère à une intégration homologue aléatoire d'un plasmide non réplicatif dans le génome de l'hôte et décrit une mutagénèse par l'intermédiaire d'un transposon. Deux transposons d'origine streptococcale, dont le transposon Tn916, ont été utilisés dans *S. thermophilus.* Des mutants ayant intégré le transposon ont été sélectionnés.

### Résumé de l'invention

L'invention se destine à fournir des nouveaux moyens pour contrôler, modifier ou restaurer la synthèse d'exopolysaccharides *in vivo* et *in vitro.*

La présente invention concerne une molécule d'ADN codant pour au moins une enzyme impliquée dans la biosynthèse d'un exopolysaccharide produit par une bactérie lactique, qui est constitué par l'assemblage de plusieurs sucres différents formant une unité répétitive présentant la structure répétée 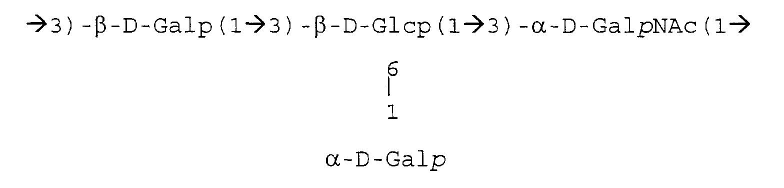 caractérisée en ce qu'elle consiste en un gène choisi dans le groupe de gènes (1) qui sont délimités dans la séquence nucléique SEQ ID NO 1 par les nucléotides 352-1803, 1807-2535, 2547-3239, 3249-3995, 4051-4731, 4898-5854, 6425-7540, 7736-8212, 8221-9192, 9285-10364, 10392-11339, 11302-12222 et 12233-13651, des séquences qui possèdent la même fonction et qui ont un taux d'identité de plus de 70% avec les séquences délimitées dans la séquence nucléotidique SEQ ID NO 1 par les nucléotides 352-1803, 1807-2535, 2547-3239, 3249-3995, 4051-4731, 4898-5854, 6425-7540, 7736-8212, 8221-9192, 9285-10364, 10392-11339, 11302-12222 et 12233-13651, et les séquences nucléotidiques qui possèdent la même fonction et qui s'hybrident dans des conditions stringentes avec les séquences délimitées dans la séquence nucléotidique SEQ ID NO 1 par les nucléotides 352-1803, 1807-2535, 2547-3239, 3249-3995, 4051-4731, 4898-5854, 6425-7540, 7736-8212, 8221-9192, 9285-10364, 10392-11339, 11302-12222 et 12233-13651.

La présente invention est également relative à un vecteur recombinant comprenant la molécule d'Adn de l'invention.

Un autre aspect de la présente invention est relatif à une protéine susceptible d'être impliquée dans la biosynthèse d'un exopolysaccharide produit par une bactérie lactique qui est constitué par l'assemblage de plusieurs sucres différents formant une unité répétitive présentant la structure répétée 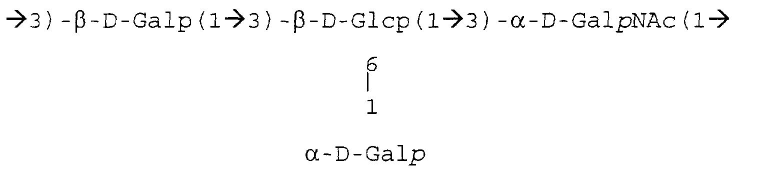 caractérisée en ce qu'elle présente la séquence en acides aminés choisie dans le groupe constitué par les séquences SEQ ID NO 2, SEQ ID NO 3, SEQ ID NO 4, SEQ ID NO 5, SEQ ID NO 6, SEQ ID NO 7, SEQ ID NO 8, SEQ ID NO 9, SEQ ID NO 10, SEQ ID NO 11, SEQ ID NO 12, SEQ ID NO 13, SEQ ID NO 14 et les séquences qui possèdent la même fonction et qui ont un taux d'identité de plus de 70% avec les séquences SEQ ID NO 2, SEQ ID NO 3, SEQ ID NO 4, SEQ ID NO 5, SEQ ID NO 6, SEQ ID NO 7, SEQ ID NO 8, SEQ ID NO 9, SEQ ID NO 10, SEQ ID NO 11, SEQ ID NO 12, SEQ ID NO 13, SEQ ID NO 14.

La présente invention est également relative à la bactérie lactique transformée par ledit vecteur.

Un autre objet de la présente invention concerne un procédé de production d'un EPS, dans lequel (1) on clone dans un vecteur un fragment d'ADN codant pour les enzymes selon l'invention, ledit vecteur comprenant en outre une séquence permettant la réplication autonome ou l'intégration dans une cellule hôte, (2) on transforme une cellule hôte par ledit vecteur, (3) puis on cultive la cellule hôte transformée dans des conditions appropriées pour la production d'un EPS.

L'invention concerne aussi un autre procédé de production d'un nouvel EPS dans lequel, (1) on clone dans un vecteur un fragment d'ADN codant pour au moins une enzyme impliquée dans la biosynthèse d'un EPS, (2) on transforme une bactérie lactique par ledit vecteur, (3) puis on cultive la bactérie lactique transformée dans des conditions appropriées pour la production d'un nouvel EPS.

La présente invention ouvre donc la possibilité d'utiliser des fragments d'ADN selon l'invention pour restaurer ou modifier la production d'EPS dans une bactérie lactique. On peut ainsi envisager d'exprimer ou de surexprimer dans une bactérie lactique l'expression des ADN selon l'invention, pour produire des EPS destinés à épaissir et rendre crémeux des boissons ou de la nourriture comme des desserts liquides, des yogourts, des soupes, des crèmes glacés, des crèmes de café, des sauces ou des mayonnaises, par exemple.

La présente invention permet aussi d'avoir des moyens nouveaux pour identifier des gènes chromosomiques de bactéries lactiques impliqués dans la biosynthèse d'EPS.

Enfin, la présente invention fournie aussi de nouvelles enzymes impliquées dans la biosynthèse de l'EPS décrit ci-dessus. Ces enzymes peuvent être ainsi avantageusement utilisées pour synthétiser ou modifier *in-vitro* un polysaccharide, comme un oligosaccharide ou un EPS, par exemple (Ichikawa Y. et al., American Chemical Society, 114, 9283-9289, 1992).

### Description des figures:

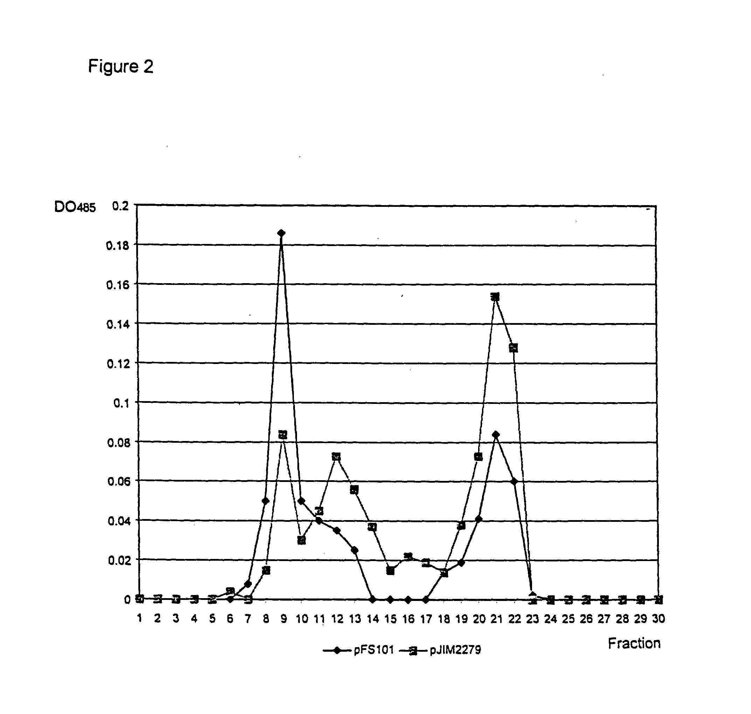

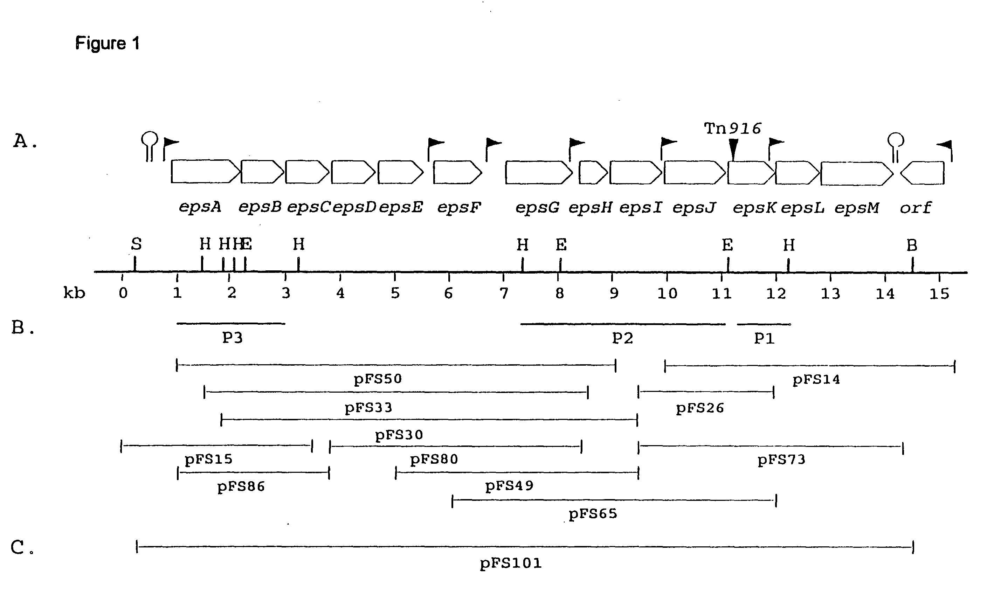

Figure 1.A. Carte physique de l'opéron impliquée dans la synthèse de l'EPS de la souche *S. thermophilus* CNCM I-1590. Les promoteurs et terminateurs sont respectivement représentés par des drapeaux et des épingles-à-cheveux. La flèche verticale indique la position du site d'insertion du transposon Tn*916.* Les flèches horizontales indiquent la présence de cadres de lectures (ORF) potentiels. Les noms des gènes correspondants aux ORFs sont indiqués en dessous des flèches. Les enzymes de restrictions sont représentées de manière abrégé (S=*Sac*I; H= *Hin*dIII; E= *Eco*RI; B=*Bam*HI).
Figure 1.B. Représentation des inserts chromosomiques de la souche CNCM I-1590, présents dans les 11 vecteurs pFS. P1, P2 et P3 indiquent la position des sondes qui sont utilisées pendant le criblage.
Figure 1.C. Représentation de l'insert génomique pFS101 comprenant tout l'operon *eps* du site de restriction *Sac*I à *Bam*HI, qui est cloné dans pJIM2279.
Figure 2. Représentation de la densité optique à 485nm des fractions de chromatographie par gel-filtration comprenant les sucres produits par la souche *Lactococcus lactis* MG1363 transformée par pFS101 ou pJIM2279. Fraction 9: 2x10⁶ Dalton (Da); fractions 11-13: 5x10⁵ Da; fractions 14-16: 7.2x10⁴ Da; fractions 17-18: 4x10⁴ Da; fraction 19 et supérieures: < 5x10³ Da.

### Description détaillée de l'invention

Dans la suite de la description, le terme "EPS" désigne un exopolysaccharide produit par une bactérie lactique qui est constitué par l'assemblage de plusieurs sucres différents formant une unité répétitive.

On désigne par les dérivés acétyl et phosphatyl, le galactose ou le glucose comprenant au moins un radical acétyl et phosphatyl aux positions C₂ à C₆ sur le cycle du sucre.

Au sens de la présente invention, on entend par "séquence homologue" toute séquence nucléique ou d'acides aminés ayant une fonction identique, ne différant des séquences selon l'invention que par la substitution, la délétion ou l'addition d'un petit nombre de bases nucléiques ou d'acides aminés, par exemple 1 à 500 paires de bases (pb) ou 1 à 150 acides aminés.

Dans ce cadre, on considèrera en particulier comme homologues deux séquences d'ADN qui, du fait de la dégénérescence du code génétique, codent pour un même polypeptide. De même, on considèrera comme homologues deux protéines fonctionnelles qui sont reconnues par un même anticorps, le rapport des valeurs d'intensité de reconnaissance des deux protéines par l'anticorps n'excédant pas 1000, de préférence 100, par exemple.

On considèrera aussi comme séquence homologue, celle qui présente plus de 70% d'homologie avec les séquences selon l'invention, en particulier plus de 80% ou 90%. Dans ce dernier cas, l'homologie est déterminée par le rapport entre le nombre de bases ou d'acides aminés d'une séquence homologue qui sont identiques à celles d'une séquence selon l'invention, et le nombre total de bases ou d'acides aminés de ladite séquence selon l'invention.

Au sens de la présente invention, on entend par "fragment qui s'hybride" tout fragment capable de s'hybrider aux fragments selon l'invention par la méthode de Southern-Blot (Sambrook et al.., Molecular Cloning, A Laboratory Manual, Cold Spring Harbor Laboratory Press, U.S.A., 1989, chapitres 9.31 à 9.58). De préférence, l'hybridation est conduite dans des conditions stringentes de manière à éviter des hybridations aspécifiques ou peu stables.

Enfin, le terme "fragment" ou "fragment d'ADN" doit être compris comme un ADN double brin d'origine chromosomique, qui peut être synthétisé, reproduit *in-vitro* par exemple par la méthode connue appelée "Polymérase Chain Reaction", ou reproduit *in-vivo* dans une bactérie du type *Escherchia coli*, *Lactococcus lactis,* ou *Streptococcus thermophilus* par exemple.

Pour sélectionner un fragment d'ADN selon la présente invention, il est possible de constituer une banque de grands fragments d'ADN d'une bactérie lactique produisant un EPS dans une bactérie lactique ne produisant pas d'EPS, puis de sélectionner le ou les clône(s) produisant un EPS. Pour cela, on digère l'ADN génomique d'une bactérie lactique produisant un EPS par une enzyme de restriction qui est spécifique d'un site de restriction relativement rare (*Bam*HI, *Sal*I, *Pst*I) ou par une digestion partielle avec *Sau*3A, par exemple. On clone le produit de digestion dans un plasmide d'expression ou d'intégration qui accepte de grands fragments (plasmide pSA3 décrit à l'exemple II), on introduit les plasmides recombinants dans la même espèce de bactérie lactique ne produisant pas d'EPS, on sélectionne au moins un clone transformé produisant un EPS, puis on identifie, on isole et on séquence classiquement le fragment d'ADN responsable de la production d'EPS.

Vu que les fragments d'ADN selon la présente invention sont susceptibles d'être de grande taille, du fait qu'ils peuvent contenir un groupe de gènes impliqués dans la biosynthèse d'EPS, on peut préférer introduire les plasmides recombinants dans la même souche de bactérie lactique dont provienne les fragments, à la différence près que cette souche a perdu la capacité de produire des EPS suite à un traitement mutagénique (traitement U.V., chimique ou par transposon).

Une alternative à la méthode décrite ci-dessus peut aussi consister à constituer une banque plasmidique de fragments d'ADN d'une souche de bactérie lactique produisant un EPS, à transformer la même souche de bactérie lactique par les plasmides incapables de s'y répliquer, à sélectionner les transformants ayant intégré un plasmide dans leur génome par recombinaison homologue (sélection par une résistance à un antibiotique, par exemple), à sélectionner les transformants ne produisant plus d'EPS, puis à isoler et séquencer les fragments d'ADN chromosomique des transformants sélectionnés qui sont adjacents au plasmide intégré. Pour cela, on peut digérer le chromosome des transformants, le liguer, puis effectuer une PCR-inverse à l'aide de sondes spécifiques du plasmide intégré ou introduire le produit de ligation dans une souche dans laquelle le plasmide recircularisé est capable de se répliquer, par exemple.

Une autre alternative à la méthode de sélection décrite ci-dessus peut aussi consister à transformer des bactéries lactiques produisant un EPS par un plasmide comprenant un transposon, à soumettre les bactéries à des conditions dans lesquelles le transposon s'excise du vecteur et s'intègre au hasard dans le génome, à sélectionner les clones de bactéries ayant perdu la capacité de produire des EPS, à isoler les fragments d'ADN génomiques desdits clones dans lesquels un transposon s'est intégré. Cette méthode est décrite plus en détail dans l'exemple I présenté ci-après.

Il faut remarquer que les méthodes de sélection décrites brièvement ci-dessus peuvent être appliquées à toutes les bactéries lactiques connues, notamment aux bactéries lactiques mésophiles comme par exemple *Streptococcus cremoris, Streptococcus lactis*, *Lactobacillus casei* subsp. *casei* et *Lactobacillus sake*, et les bactéries lactiques thermophiles comme par exemple *Streptococcus thermophilus*, *Lactobacillus delbruecki* subsp. *bulgaricus* et *Lactobacillus helveticus.* A cet effet, l'homme du métier dispose de techniques de transformation pour chaque espèce de bactérie lactique, et en particulier pour *Lactobacillus delbruecki* subsp. *bulgaricus* (Sasaki Y. *et al*., FEMS Microbiology Reviews, 12, Fourth Symposium on Lactic Acid Bacteria, Noodwijkerhout, The Netherlands, Sept 1993).

De plus, les méthodes de sélection décrites ci-dessus permettent le plus souvent d'isoler seulement une partie d'un gène ou d'un groupe de gènes impliqués dans la biosynthèse d'un EPS. Néanmoins, l'homme du métier peut facilement identifier la partie restante du gène ou du groupe de gènes en sélectionnant dans une banque chromosomique, à l'aide de sondes nucléiques basées sur un fragment isolé, un ou plusieurs clones renfermant la partie restante, par exemple (voir l'exemple I.6)

On a pu ainsi caractériser une séquence d'ADN de 15,2 kb de la souche *Streptococcus thermophilus* déposée le 7 juin 1995, auprès de la Collection Nationale de Culture de Microorganisme (C.N.C.M.), Institut Pasteur, 28 rue du Dr Roux, 75724 Paris cedex 15, France, où elle a reçu le numéro de dépôt CNCM I-1590. Par ailleurs, cette souche Gram-positif présente au microscope un aspect de coques non flagellées formant des chaînettes. Cette souche ne fait pas de spores et elle est anaéorobe facultative.

Cette séquence de 15,2kb comprend des gènes codant pour des enzymes nouvelles impliquées dans la biosynthèse d'un EPS ayant la structure répétée

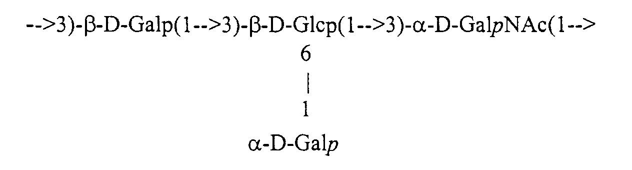

Les nucléotides 648 à 15250 de cette séquence de 15,2kb sont représentés dans la séquence SEQ ID NO:1 donnée dans la liste de séquence ci-après. 13 gènes complets sont délimités dans la séquence nucléique SEQ ID NO:1 par les nucléotides 352-1803, 1807-2535, 2547-3239, 3249-3995, 4051-4731, 4898-5854, 6425-7540, 7736-8212, 8221-9192, 9285-10364, 10392-11339, 11302-12222, et 12233-13651.

On a pu montrer que tout ou partie de la séquence SEQ ID NO:1 kb permet, suite à une transformation, de restaurer une biosynthèse d'EPS dans une cellules hôte, comme une bactérie lactique mésophile ou thermophile qui initialement n'en produisait pas, notamment dans un *Streptococcus* ou un *Lactococcus.* A titre d'exemple, la séquence d'ADN selon l'invention peut ainsi être utilisée pour restaurer la production d'EPS dans un mutant de la souche *S. thermophilus* CNCM I-1590 n'en produisant plus (mutant naturel ou issu d'une mutagenèse).

Pour restaurer la biosynthèse d'un EPS, on peut intégrer tout ou partie de la séquence SEQ ID NO:1 comprenant au moins un des gènes précités dans une cellule hôte au moyen du procédé décrit dans EP564966, ledit procédé étant incorporé par référence dans l'enseignement de la présente invention. En résumé, ce procédé permet de pouvoir (1) transformer la cellule hôte avec un plasmide donneur qui ne s'y réplique pas, ledit plasmide comprenant ledit fragment intégré fonctionnellement (le cadre de lecture est conservé) dans une partie d'un opéron issu de la cellule hôte; (2) identifier les transformants comprenant intégré la totalité du plasmide; (3) sélectionner des transformants comprenant uniquement intégré dans le chromosome le fragment selon l'invention, les autres séquences du plasmide s'étant excisé du chromosome; (4) et cultiver les transformants sélectionnés dans des conditions appropriées pour la production d'un EPS.

On peut noter que ce procédé permet de ne pas utiliser des séquences promoteur et d'activation traductionnelle fonctionnels. De plus, les conditions de culture appropriées pour la production d'EPS sont à la portée de l'homme du métier, qui peut utiliser des milieux de culture standards, et choisir le pH, la température et l'agitation du milieu optimum selon la souche utilisée.

On peut aussi choisir de cloner tout ou partie de la séquence SEQ ID NO:1 comprenant au moins un des gènes précités dans un plasmide d'expression autoréplicatif en aval de séquences promoteur et d'activation traductionnelle fonctionnels, et le cas échéant en amont d'un terminateur, puis de transformer une cellule hôte par le plasmide recombinant.

Par ailleurs, on peut observer que l'EPS produit par une cellule hôte transformée par la séquence SEQ ID NO:1, par exemple un *Lactococcus lactis* ne produisant pas initialement un EPS, peut être différent de l'EPS qui devrait être normalement synthétisé par les enzymes recombinantes, en l'occurence l'EPS produit par la souche CNCM I-1590. L'utilisation de tout ou partie de la séquence de 15,2 kb peut donc permettre la création de variants de l'EPS décrit ci-dessus.

De même, on a pu montrer que tout ou partie de la séquence SEQ ID NO:1 peut aussi permettre, suite à une transformation, de modifier la structure répétée d'un EPS produit initialement par une cellule hôte, par exemple par une bactérie lactique mésophile ou thermophile, notamment un *Streptococcus* ou un *Lactococcus.*

Ces observations ouvrent ainsi la possibilité de réaliser une méthode originale de production d'un nouvel EPS, dans laquelle (1) on clone dans un vecteur un fragment d'ADN codant partiellement ou totalement pour au moins une enzyme impliquée dans la biosynthèse d'un EPS; (2) on transforme des bactéries lactiques par le vecteur recombinant; (3) on sélectionne le cas échéant une bactérie lactique produisant un nouvel EPS; (4) puis on cultive la bactérie lactique transformée dans des conditions appropriées pour la production d'un nouvel EPS. De préférence le vecteur code pour les protéines selon l'invention. De plus la bactérie lactique peut produire un autre EPS que celui synthétisé par les protèines codées par ledit vecteur.

En particulier, on clone dans un vecteur d'intégration un fragment d'ADN codant partiellement pour au moins une enzyme impliquée dans la biosynthèse d'un premier EPS, on introduit le vecteur recombinant dans des bactéries lactiques mésophiles ou thermophiles, pouvant le cas échéant produire un deuxième EPS par l'intermédiaire d'un ou plusieurs gènes chromosomiques ou plasmidiques, on isole les bactéries ayant intégrés dans leur chromosome le vecteur d'intégration, puis on sélectionne celles qui produisent un nouvel EPS à cause de l'inactivation d'un ou plusieurs gènes impliqués dans la biosynthèse du deuxième EPS. De préférence, le premier et le deuxième EPS sont identiques, et on choisit un fragment d'ADN codant partiellement (au moins 15 paires de bases) pour au moins une enzyme impliquée dans l'adjonction d'un sucre sur la chaine latérale de l'unité répétitive ou dans la modification d'un sucre comme une sulpho-, phosphoryl- ou acétyl-transférase, par exemple.

De même, on peut cloner dans un vecteur d'expression réplicatif un fragment d'ADN codant totalement pour au moins une enzyme impliquée dans la biosynthèse d'un premier EPS, on peut introduire le vecteur recombinant dans des bactéries lactiques mésophiles ou thermophiles, pouvant le cas échéant produire un deuxième EPS par l'intermédiaire d'un ou plusieurs gènes chromosomiques ou plasmidiques, on peut isoler les bactéries renfermant le vecteur réplicatif, puis on peut sélectionner celles qui produisent un nouvel EPS à cause de l'expression d'un ou plusieurs gènes impliqués dans la biosynthèse du premier EPS. De préférence, on choisit des fragments d'ADN codant pour des enzymes impliquées dans la modification d'un sucre comme une sulpho-, phosphoryl- ou acétyl-transférase par exemple, ou dans l'adjonction à l'unité répétitive d'un sucre comme une glucosyl- ou une galactosyl-transférase, par exemple.

De préférence, on utilise totalement ou partiellement au moins un des gènes portés par la séquence SEQ ID NO:1. On peut aussi utiliser au moins un gène plasmidique de bactéries lactiques mésophiles impliqué dans la biosynthèse d'un EPS (gène que l'on peut séquencer à partir de plasmides connus).

Enfin, le vecteur recombinant peut être tout fragment d'ADN, simple ou double brin, linéaire ou circulaire, d'expression ou d'intégration, et comprenant un une séquence d'ADN selon l'invention notamment tout ou partie de la séquence SEQ ID NO:1. Dans le cas où le procédé décrit dans EP564966 n'est pas utilisé, il faut veiller à ce que le vecteur puisse exprimer l'ADN selon l'invention par des séquences nucléiques adaptées (promoteur; site d'attachement du ribosome; codon préféré), et le cas échéant à ce qu'il comprenne une ou plusieurs origines de réplication de diverses bactéries, notamment d'*Escherichia coli* et/ou d'un *Streptococcus*, par exemple.

L'invention concerne aussi les nouvelles enzymes codées par les gènes de la séquence SEQ ID NO:1, notamment les séquence qui leur sont homologues On peut ainsi envisager de les utiliser pour modifier ou synthétiser *in-vitro* un oligosaccharide ou un polysaccharide comme un EPS, par exemple. Pour cela, il est préférable de purifier au moins une de ces enzymes, en surexprimant classiquement leur gène dans une bactérie et en les isolant classiquement, par précipitation et/ou chromatographie du milieu de culture, par exemple.

Un autre objet de la présente invention concerne une bactérie lactique comprenant, intégré dans son chromosome ou par le moyen d'un plasmide réplicable, une séquence d'ADN selon l'invention. De préférence, la séquence comprend au moins un des gènes de la séquence SEQ ID NO:1.

L'invention concerne aussi toute utilisation de fragments de la séquence SEQ ID NO:1 ou de fragments du brin complémentaire de cette séquence, d'au moins 15 paires de bases, comme amorce pour faire une PCR ou comme sonde pour détecter *in-vitro* ou inactiver *in-vivo* des gènes de bactéries lactiques impliqués dans la biosynthèse d'un EPS. Cette limite inférieure est arbitrairement fixée du fait que les petits fragments s'hybridant spécifiquement ont généralement une longeur de 15-25 pb.

La présente invention est décrite plus en détail ci-après à l'aide du complément de description qui va suivre, qui se réfère à des exemples d'obtention de fragments d'ADN, de plasmides recombinants et de bactéries transformées selon l'invention. Ces exemples sont précédés d'une description des milieux de culture. Il va de soi, toutefois, que ces exemples sont donnés à titre d'illustration de l'objet de l'invention dont ils ne constituent en aucune manière une limitation. La manipulation de l'ADN, le clonage et la transformation de cellules bactériennes sont, en l'absence de précisions contraires, effectués selon les protocoles décrits dans l'ouvrage de Sambrook *et al.* cité plus haut. Les pourcentages sont donnés en poids, sauf indication contraire.

### Milieux: (rajouter 1,5% de Bacto-agar pour un milieu solide)

- M17 (Difco,USA):tryptone 0,5%, soytone 0,5%, viande hydrolysée 0,5%, extrait de levure 0,25%, acide ascorbique 0,05%, sulphate de magnésium 0,025%, disodium-beta-glycérophosphate 1,9% et de l'eau.
- LM17: milieu M17 comprenant 1% de lactose.
- GM17: milieu M17 comprenant 1% de glucose.
- MSK: lait écrémé (poudre reconstituée à 10%) comprenant 0,1% d'extrait de levure.
- MAM: lait écrémé (poudre reconstituée à 10%) comprenant 10% d'un mélange d'acides aminés (495 mg/l Ala, 343 mg/l Arg, 682 mg/l Asp, 59 mg/l Cys, 1229 mg/l Glu, 759 mg/l Gly, 153 mg/l His, 215 mg/l Iso, 470 mg/l Leu, 565 mg/l Lys, 122 mg/l Met, 255 mg/l Phe, 436 mg/l Pro, 68 mg/l Ser, 170 mg/l Thr, 61 mg/l Try, 304 mg/l Val ajusté à pH5).
- HJL: tryptone 3%, extrait de boeuf 0,2%, extrait de levure 1%, lactose 1% et KH₂PO₄pH 6,5 0,5%.
- Rouge de Ruthénium: extrait de levure 0,5%, lait en poudre écrémé 10%, sucrose 1%, agar 1,5% et 0,08g/l de rouge de ruthénium (voir FR2632968).

### Exemple I: clonage d'un fragment d'ADN de la souche S. thermophilus Sfi6

I.1. Sélection d'une souche *S. thermophilus* productrice d'EPS: on cultive les souches de bactéries lactiques de la collection Nestlé dans un milieu liquide HJL et on en étale des dilutions sur un milieu solide Rouge de Ruthénium. Les souches productrices d'EPS demeurent de couleur blanche car les EPS empêchent le colorant de teinter leur paroi cellulaire. Par contre, les souches non-productrices se colorent en rouge du fait de l'affinité du colorant pour le peptidoglycane de leur paroi cellulaire.

On a ainsi sélectionné parmi les bactéries lactiques productrices d'EPS la souche S. *thermophilus* Sfi6, qui a reçu le numéro de dépôt CNCM I-1590 et que l'on désignera dans la suite des exemples par l'expression "souche Sfi6".

I.2 .Structure répétée de l'EPS: la structure de l'EPS produit par la souche Sfi6 a été publiée par Doco *et al*. (Carbohyd.Res., 198, 313-321, 1995). Cet EPS présente la composition Glc:Gal:GalNac=1:2:1, et l'unité tétrasaccharidique répétée:

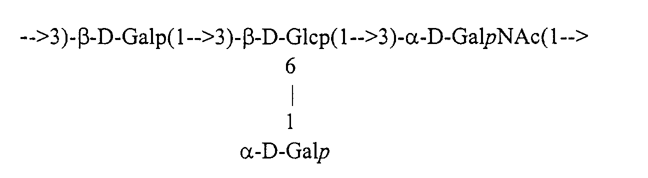

I.3. Mutagenèse par le transposon Tn*916*: on rend la souche Sfi6 résistante à la streptomycine en la cultivant par des transferts répétés dans un milieu HJL supplémenté par des teneurs croissantes de 20 à 2000µg/ml de streptomycine, puis en sélectionnant les souches devenues naturellement résistantes.

On conjugue la souche Sfi6 résistante à la streptomycine et la souche *Enterococcus faecalis* JH2-2 qui possède un plasmide pAM180 portant le transposon Tn*916 (Tn916* est connu pour porter un gène de résistance à la tetracycline; Gawron *et al*., Nature, 300, 281-283, 1982). Pour cela, on mélange à 1ml d'une culture d'une nuit dans un milieu M17 à 37°C de la souche *E. faecalis* JH2-2, 10ml d'une culture d'une nuit dans un milieu HJL à 42°C de la souche Sfi6, on centrifuge les cellules et on les resuspend dans des tubes comprenant 100µl de milieu HJL, on dépose la suspension sur un milieu solide LM17 que l'on incube à 37°C pendant 20h, on récupère les cellules par grattage et on les resuspend dans des tubes de 10 ml de milieu liquide HJL, on incube les tubes à 42°C pendant 4h en les agitant de temps en temps, puis on étale des dilutions des cultures sur un milieu LM17 solide supplémenté de 2,5µg/ml de tetracycline et 2000µg/ml de streptomycine.

En réalisant 20 conjugaisons en parrallèles (mutations indépendantes), on a pu ainsi sélectionner 2x10⁴ transconjugants résistants à la tetracycline et à la streptomycine.

I.4. Sélection de mutants de la souche Sfi6 ne produisant plus d'EPS [phénotype EPS(-)]: on transfert les transconjugants résistants sur le milieu solide Rouge de Ruthénium supplémenté par 2,5µg/ml de tetracycline et 2000µg/ml de streptomycine. Environ 10% des transconjugants forment des colonies rouges EPS(-). On sélectionne ensuite environ 800 colonies rouges que l'on cultive une nuit dans des plaques de microtitration comprenant 200µl de milieu HJL supplémenté de 2,5µg/ml de tetracycline. On cultive ensuite 100µl de la culture HJL dans 1ml d'un lait MSK. Environ, 25% des colonies rouges testées présentent un phénotype EPS(-) stable dans le lait (le lait n'est pas épais et filant, et l'analyse du surnageant de culture ne révèle pas d'EPS). Les autres colonies rouges présentent un phénotype EPS(+) ou retrouvent le phénotype EPS(+) après plusieurs sous-cultures dans le lait.

En conclusion, les mutants stables EPS(-) ont perdu leur capacité à produire des EPS à cause de l'intégration du transposon *Tn916* dans un gène chromosomique impliqué dans la biosynthèse des EPS. En effet, les mutants stables EPS(-) peuvent retrouver un phénotype EPS(+) lorsqu'on les cultive dans un milieu de croissance dépourvu de tetracycline (excision et perte du transposon).

I.5 Caractérisation de mutants stables EPS(-): on analyse environ 100 mutants stables par Southern-blot d'une préparation d'ADN chromosomique des mutants, digérée par *Hin*dIII, et hybridation du filtre de Southern-blot avec le gène *tetM* radioactif (code une résistance à la tetracycline) provenant du plasmide pIC182 (Hill *et al*., Applied and Env. Micro., 54, 1230-1236, 1988). Environ 85% des mutants analysés présentent une bande majoritaire identique correpondant à un locus appellé "locusA". On peut remarquer pour certains des autres mutants deux autres bandes majoritaires (locus B et C) correspondant à des locus connus impliqués dans la biosynthèse de la paroi cellulaire (publication en préparation).

I.6 Caractérisation du locus A: les régions chromosomiques proches du transposon *Tn916* intégré peuvent être isolées par une PCR-inverse. Pour cela, on digère classiquement 1µg d'une préparation d'ADN chromosomique d'un mutant choisi arbitrairement (mutant n°1) par *Hin*dIII pendant 4h, on extrait l'ADN au phénol/chloroforme, on le dilue dans 720µl d'eau, on chauffe l'ADN dilué à 56°C pendant 5 min, on refroidit l'ADN sur de la glace, on lui ajoute 80µl d'un tampon de ligation 10 fois concentré et 5 unités d'une T4-ligase (Boehringer-Manheim), on l'incube à 12°C pendant 16 h, on le chauffe à 70°C pendant 15 min pour inactiver la ligase, puis on le concentre dans un volume de 100µl par plusieurs extractions successives dans du butanol. On ajoute alors dans un dispositif de PCR 10µl du mélange de ligation, 100pmol d'amorces, 15mM de dNTPs, 10µl de tampon et 0,2 unité de Super-Taq polymerase (Stehlin GmBH). Les amorces nucléiques (ou primers) sont choisies à partir de la séquence connue du transposon Tn*916.*

En utilisant les amorces ayant la séquence SEQ ID NO:15 et SEQ ID NO:16 on a pu isoler par PCR un fragment de 1kb. De plus, en utilisant les amorces SEQ ID NO:17 et SEQ ID NO:18 on a pu isoler un fragment de 4kb (voir la liste de séquences ci-après).

Un troisième fragment de 0.8kb peut être aussi isolé du mutant n°1, en réalisant une seconde PCR-inverse à partir de son ADN chromosomique digéré par R*sa*I et à l'aide des amorces ayant la séquence SEQ ID NO:18 et SEQ ID NO:19 (voir la liste de séquence ci-après).

Les fragments de 1kb et de 0.8kb ont été clonés dans le plasmide linéarisé pGEMT (Promega, USA). Le séquencage de ces fragments par la méthode des didéoxynucléotides (kit f-mol® DNA Sequencing System, Promega) montre deux séquences qui, en se recoupant, couvrent trois cadres de lectures ouvertes (ORFs) correpondants aux nucléotides 9933 à 11643 de la séquence SEQ ID NO:1.

Les fragments de 1kb et 4kb ont également été utilisés pour cribler une banque λ-ZAP Express (Stratagene, USA) renfermant des fragments d'ADN de la souche Sfi6. Pour cela, selon les recommandations du fournisseur on digère partiellement une préparation d'ADN dudit mutant par *Sau*3A, on sépare les fragments par une électrophorèse sur gel d'agarose, on coupe du gel les bandes correspondantes à des fragments de 5 à 12kb, on élue l'ADN, puis on le ligue au vecteur λ-ZAP Express préalablement digéré par *Bam*HI. On encapside *in-vitro* le produit de ligation à l'aide du système GigagoldIII (Stratagene), on mélange ensuite les phages avec des *Escherichia coli* XL1Blue (Stratagene) selon les recommandations du fournisseur, puis on étale le mélange sur boîte de Petri. On analyse ensuite les plaques recombinantes par hybridation de leur ADN transféré sur une membrane Hybond-N (Amersham Life Sciences, UK) avec les fragments de 1kb et 4kb préalablement rendus radioactifs (kit Random Primed DNA Labeling, Boehringer-Manheim).

Parmi 3000 plaques recombinantes, on a pu sélectionner par hybridation environ 20 plaques positives, desquelles on a ensuite isolé les vecteurs λ-ZAP Express, puis excisé les vecteurs pCMV renfermant un insert chromosomique (voir les recommandations du fournisseur Stratagene). Ces vecteurs recombinants sont appelés dans la suite des exemples "pFS".

On a ensuite séquencé les inserts chromosomiques de 11 vecteurs pFS (kit f-mol® DNA Sequencing System), à savoir les vecteurs pFS14, pFS15, pFS26, pFS30, pFS33, pFS49, pFS50, pFS65, pFS73, pFS80 et pFS86 (voir figure 1.B) qui comprennent respectivement des fragments correspondant aux nucléotides de la séquence SEQ ID NO:1, 9314-14602, 1-3159, 7988-11253, 1702-7991, 1361-7229, 4400-8477, 648-7676, 5997-11253, 8474-13489, 3550-7229, et 648-1702.

En recoupant les séquences nucléiques des différents inserts chromosomiques, on a pu ainsi caractériser une séquence de 15,2kb correspondant au locus A de la souche Sfi6 (voir figure 1.A). Les nucléotides 648 à 15250 de cette séquence de 15,2kb sont représentés dans la séquence SEQ ID NO:1.

### I.7. Analyse de la séquence SEQ ID NO:1:

La séquence SEQ ID NO:1 comprend la totalité de l'opéron *eps* de la souche Sfi6. Cette séquence comprend 13 ORFs complets, dans la même orientation, que l'on appelle *eps A*, *B*, *C*, *D*, *E*, *F*, *G*, *H*, *I*, *J*, *K*, *L*, *M* (voir figure 1.A). Cette séquence comprend en outre 1 ORF complet à l'extrémité 3' de la séquence, qui est codé par le brin complémentaire. Cet ORF, appellé *orf*Z, marque probablement la fin de l'opéron du fait de son orientation inverse par rapport aux autres ORFs.

La comparaison des séquences en acides aminés codées par les 13 premiers ORFs avec celles de protéines présentes dans la banque de donnée Swiss-Prot, à l'aide des logiciels FASTA, PEPPLOT et PILEUP de GCG-softwear, Wisconsin, USA, permet de déduire la fonction des 13 protéines codées par l'opéron *eps.* Les résultats sont présentés ci-après.

L'ORF *epsA* (nucléotides 352-1803) code pour une protéine EpsA (SEQ ID NO:2) ayant 26,4% d'identité avec la protéine LytR de *Bacillus subtilis* qui est impliquée dans la régulation de l'autolysine *N*-acetylmuramoyl-L-alanine (Lazaveric *et al*., J. Gen. Microbiol., 138, 1949-1961, 1992). EpsA est donc probablement une protéine de régulation de l'opéron *eps.* Par ailleurs, puisqu'un ORF de régulation d'un opéron est généralement trouvé en amont des autres ORFs, le gène *epsA* est probablement le premier gène de l'opéron *eps.* Ceci est confirmé par le fait qu'un terminateur est trouvé aux nucléotides 230-252, un promoteur aux nucléotides 274-302, et un site d'attachement des ribosomes aux nucléotides 340-345 de la séquence SEQ ID NO:1.

Le gène *epsB* (nucléotides 1807-2535) code pour une protéine EpsB (SEQ ID NO:3) ayant 67,5% d'identité avec la protéine CpsA de *Streptococcus agalactiae* et 30% d'identité avec la protéine CapC de *Staphylococcus aureus* (Rubens *et al*., Mol. Microbiol., 8, 843-885, 1993; Lin *et al*., J. Bacteriol., 176, 7005-7016, 1994). La fonction précise de ces gènes est encore inconnue, en dehors du fait qu'ils sont essentiels pour la synthèse de la capsule qui est constituée de polysaccharides accrochés aux phospholipides de la membrane externe des bactéries.

Le gène *epsC* (nucléotides 2547-3239) code pour une protéine EpsC (SEQ ID NO:4) ayant 52% d'identité avec la protéine CpsB de *Streptococcus agalactiae* qui est impliquée dans la synthèse de la capsule (Rubens *et al*.). EpsC a aussi 23% d'identité, 49% de similarité, et un profil d'hydrophobicité comparable à celui des protéines CLD de *Salmonella typhimurium*, *Salmonella enterica* et *Escherichia coli* (Batchelor *et al*., J. Bacteriol., 174, 5228-5236, 1992; Bastin *et al*., Mol. Microbiol., 7, 725-734, 1993). Il faut remarquer que les protéines CLD sont impliquées dans le contrôle de la longeur des chaînes de polysaccharides lors de leur biosynthèse.

Le gène *epsD* (nucléotides 3249-3995) code pour une protéine EpsD (SEQ ID NO:5) ayant 60,5% d'identité avec la protéine CpsC de *Streptococcus agalactiae,* ayant 34,5% d'identité avec la protéine CapA de *Staphylococcus aureus*, et ayant 33% d'identité avec la protéine ExoP de *Rhizobium meliloti* (Rubens *et al*.; Lin *et al*.; Becker *et al*., Mol. Gen. Genet., 241, 367-379, 1993). La protéine ExoP est une protéine de membrane qui est impliquée dans la translocation d'EPS et/ou de précurseurs d'EPS.

Le gène *epsE* (nucléotides 4051-4731) code pour une protéine EpsE (SEQ ID NO:6) présentant des homologies significatives avec de nombreuses protéines ayant une activité galactosyl-transférase (Rubens *et al*.). Ce gène code donc probablement pour une galactosyl-transférase.

On peut remarquer que les gènes *epsB*, *C*, *D*, *E* de *S. thermophilus* Sfi6 sont similaires à ceux de l'opéron de *S. agalactiae* comprenant les gènes *cpsA*, *B*, *C*, *D* (Rubens *et al*.). De plus, ils sont organisés de la même façon. Bien que les polysaccharides de capsule et l'EPS des deux souches soient très différents, ceci indique qu'une région chromosomique a été probablement tranférée entre ces deux espèces.

Le gène *epsF* (nucléotides 4898-5854) code pour une protéine EpsF (SEQ ID NO:7) ayant respectivement 24,5% et 23% d'identité avec les protéines CapH et CapM de *S. mutans* qui sont impliquées probablement en tant que glycosyl-transférases dans la biosynthèse de la capsule (Lin *et al*.).

Le gène *epsG* (nucléotides 6425-7540) code pour une protéine EpsG (SEQ ID NO:8) ayant 20,5% d'identité et 50% de similarité avec la N-acétylglucoseamine-transférase de *Salmonella typhimurium* LT2 qui est impliquée dans la biosynthèse du polysaccharide LPS de la membrane externe (Mac Lachlan *et al*., J. Bacteriol., 173, 7151-7163, 1991). Du fait qu'une *N*-acétylglucosamine n'est pas impliquée dans la biosynthèse de l'EPS de la souche Sfi6 (il n'y a pas de glucose acetylé), le gène *epsG* code probablement pour une glucosyl-transférase, une *N*-acétylgalactosyl-transférase, ou une *N*-acétylglucosyl-transférase ayant une activité *N*-acétylglucosamine-épimérase.

Le gène *epsH* (nucléotides 7736-8212) code pour une protéine EpsH (SEQ ID NO:9) ayant de fortes homologies avec des acétyl-transférases NodL-LacA-CysE (Downie *et al*., Mol. Microbiol. 3, 1649-1651, 1989). De ce fait la protéine EpsH pourrait être une acétyl-transférase impliquée dans la biosynthèse de la N-acétylgalactoseamine de l'EPS.

Le gène *epsI* (nucléotides 8221-9192) code pour une protéine EpsI (SEQ ID NO:10) ayant 24% d'identité avec une protéine, codée par un l'ORF RfbV du cluster *rfb* de *Salmonella typhimurium*, qui est probablement une glycosyl-transférase (Jiang *et al*.; Liu *et al*., J. Bacteriol., 177, 4084-4088, 1995).

Le gène *epsJ* (nucléotides 9285-10364) code pour une protéine EpsJ (SEQ ID NO:11) ayant 20% d'identité et un profil d'hydrophobicité comparable à celui d'une protéine d'un ORF du cluster *rfb* de *Salmonella enterica* qui est lui-même similaire à une polymérase de l'antigène O des salmonelles du groupe B et C2 (Lee *et al*., J. Gen, Microbiol., 138, 1843-1855, 1992; Morona *et al*., J. Bacteriol. 176, 733-747, 1994). Le gène *epsJ* pourrait donc coder une EPS-polymérase qui polymériserait l'unité tétrasaccharide de l'EPS.

Le gène *epsK* (nucléotides 10392-11339) code pour une protéine EpsK (SEQ ID NO:12) ayant 18% d'identité et 42% de similarité avec la protéine, codée par le gène *lipB* de *Neisseria meningitidis*, qui est impliquée dans la biosynthèse de la capsule en accrochant des polysaccharides aux phospholipides de la membrane externe (Frosch *et al*., Mol. Microbiol., 8, 483-493, 1993). Sachant que les *S. thermophilus* n'ont pas de membrane externe (Gram-positif), le gène *epsK* pourrait donc coder une enzyme impliquée dans l'accrochage des EPS aux phospholipides de la membrane cellulaire, qui de concert avec un transporteur d'EPS (probablement EpsC et EpsD) et une enzyme qui détache les EPS, participerait au transport de l'EPS à travers la membrane (modèle en accord avec celui présenté par Frosch *et al*.).

Par ailleurs, on peut remarquer que le transposon Tn*916* est intégré dans le gène *epsK* du mutant n° 1 utilisé pour identifier l'opéron *eps* (voir le point I.6 ci-dessus), entre les nucléotides 10540-10541 de la séquence SEQ ID NO:1.

Le gène *epsL* (nucléotides11302-12222) code pour une protéine EpsL (SEQ ID NO:14) qui ne présente aucune homologie avec des protéines connues. Les 38 premiers nucléotides sont couverts par l'extrémité 3' de *epsK,* ce qui laisse supposer une expression coordonnée des deux protéines, et une activité de la protéine EpsL dans le transport membranaire de l'EPS.

Le gène *epsM*(nucléotides 12233-13651) code pour une protéine EpsM (SEQ ID NO:13) qui ne présente aucune homologie avec des protéines connues de la banque de données Swiss-prot. Ce gène est certainement impliqué dans la biosynthèse de l'EPS de la souche Sfi6 car il n'y a pas, en amont, un promoteur spécifique pour ce gène.

Le gène *orfZ* (13732-14305 sur le brin complémentaire) est présent en orientation inverse par rapport au reste des ORFs de l'opéron *eps.* De ce fait, il n'est probablement pas impliqué dans la biosynthèse de l'EPS de la souche Sfi6. De plus, il ne présente aucune homologie avec des protéines connues de la banque de données Swiss-prot.

En conclusion, les inserts chromosomiques isolés des 11 vecteurs pSF (voir le point I.6 ci-dessus) couvrent une région chromosomique de la souche *S*. *thermophilus* Sfi6 qui est manifestement impliquée dans la biosynthèse de l'EPS. On a pu ainsi identifier 13 gènes complets qui comprennent en amont un promoteur délimitant le début de l'opéron *eps.*

### Exemple II: inactivation du gène epsJ

On inactive par recombinaison homologue le gène *epsJ* de l'opéron *eps* pour confirmer son importance dans la biosynthèse de l'EPS.

Pour cela, on isole un fragment *Dra*I-*Sal*I du plasmide pGEMT renfermant le fragment de PCR de 0.8 kb (voir l'exemple I.6 ci-dessus), on le ligue dans le plasmide thermosensible pSA3 (Dao *et al*., Appl. Environ. Microbiol., 49, 115119, 1985) préalablement digéré par *Eco*RV et *Sal*I, on transforme la souche *E. coli* XL1-blue par le produit de ligation, on sélectionne des transformants, on isole un plasmide recombinant, puis on transforme par électroporation la souche *S. thermophilus* Sfi6 avec le plasmide recombinant au moyen d'une méthode adaptée de celle décrite par Slos *et al.* (Appl. Environ. Microbiol., 57, 1333-1339, 1991). On resuspend les cellules soumises à une décharge de 2,1kV, 25µF et 400Ω dans 1ml de milieu HJL que l'on incube 4h à 37°C (température permissive), on étale les cellules sur un milieu solide LM17 supplémenté de 2,5µg/ml d'erythromycine que l'on incube 16h à 37°C, puis on sélectionne les colonies transformées qui survivent. On incube ensuite les colonies sélectionnées dans 2 ml de milieu HJL supplémenté de 2,5µg/ml d'erythromycin jusqu'à ce que la densité optique à 600nm (DO₆₀₀) de la culture atteigne 0,2, on soumet la culture à 45°C jusqu'à ce que la DO₆₀₀ atteigne 1.0 (le plasmide ne se réplique plus), puis on étale des dilutions de la culture sur un milieu LM17 solide supplémenté de 2,5µg/ml d'erythromycine que l'on incube 12h à 45°C.

Les colonies qui survivent ont intégré dans le gène *epsJ* le plasmide pSA3 recombinant. Ceci peut être vérifié par Southern-Blot d'une préparation d'ADN chromosomique des colonies survivantes digérée par *Eco*RI (coupe une seule fois dans pSA3), et hybridation du filtre de Southern-Blot avec le fragment radioactif précité *Dra*I-*Sal*I. Les colonies ayant intégré le plasmide pSA3 présentent deux bandes sur le filtre de Southern-Blot. De plus, les colonies ayant intégré dans *epsJ* le plasmide pSA3 recombinant présentent un phénotype EPS(-) sur un milieu solide Rouge de Ruthénium, et ont perdu leur caractère filant dans un lait MSK (voir l'exemple I.4 ci-dessus).

### Exemple III: inactivation des gènes eps A, B, C, D, E, F, G, H, I, K, L, M

On a montré aux exemples I et II que l'inactivation des gènes *epsK* et *epsJ*, par insertion d'un transposon ou d'un plasmide intégratif, interrompt la biosynthèse d'EPS dans la souche Sfi6.

De même, on peut inactiver par recombinaison homologue les autres gènes de l'opéron *eps* de la souche Sfi6, et observer ainsi une interruption de la biosynthèse d'EPS. Pour cela, on amplifie par PCR un fragment d'un ORF provenant d'un des 11 vecteurs pFS décrits à l'exemple I.6 ci-dessus. On le clone dans le plasmide pSA3, puis on le transforme et on l'intègre à la souche Sfi6 dans les mêmes conditions que celles décrites à l'exemple précédent.

### Exemple IV: restauration de la production d'EPS

On coupe par *Eco*RI pFS30, on sépare les fragments, on ligue le fragment de 5.5 kb à pFS14 préalablement digéré par *Eco*RI, on transforme des cellules XL1-blue par le produit de ligation, on sélectionne des clones transformés présentant une bonne orientation des inserts, on isole un plasmide appellé pFS30-14, on ligue un fragment *Eco*RI central de pFS65 à pFS30-14 préalablement coupé par *Eco*RI, on transforme des cellules XL1-blue par le produit de ligation, puis on sélectionne des clones transformés présentant une bonne orientation des inserts. Le plasmide recombinant résultant, appellé pFS30-65-14, comprend les nucléotides 1702 à 14602 de la séquence SEQ ID NO:1.

On coupe ensuite pFS30-65-14 par *Sal*I et *Sma*I, on sépare le fragment de 12.9 kb, on le ligue à pSA3 préalablement coupé par *Eco*RV et *Sal*I, on transforme des cellules XL1-blue par le produit de ligation, on sélectionne des clones transformés, et on isole des plasmides pSA3 recombinants.

On transforme par électroporation la souche *S. thermophilus* CNCM I-1292 déposée le 29 mars 1993 par les plasmides pSA3 recombinants. Cette souche Gram-positive présente au microscope un aspect de coques non flagellées formant des chaînettes, elle ne fait pas de spores, elle est anaéorobe facultative, elle ne produit pas d'EPS, et elle présente dans son génome 1000 pb correspondant à l'extrémité 5' de l'operon *eps.* Le plasmide pSA3 recombinant peut donc s'intégrer dans le génome de la souche CNCM I-1292. Certains des clones transformés présentent un phénotype EPS(+) sur un milieu solide Rouge de Ruthénium, et un caractère filant dans un lait MSK.

### Exemple V restauration de la production d'EPS

On digère le chromosome de la souche Sfi6 par des enzymes qui ne coupent pas dans la séquence SEQ ID NO:1 (*Bam*HI, *Sal*I, *Nru*I, *Stu*I), on sépare le produit de digestion sur un gel d'agarose, on élue les bandes de 15-25 kb, on les ligue dans pSA3 préalablement coupé par une enzyme de restriction appropriée, on transforme par électroporation la souche *S. thermophilus* CNCM I-1292, puis on sélectionne des transformants par transferts des colonies sur un filtre suivi d'une hybridation de leur ADN avec l'insert de pFS14 rendu préalablement radioactif. Certains des clones transformés présentent un phénotype EPS(+) sur un milieu solide Rouge de Ruthénium, et un caractère filant dans un lait MSK.

### Exemple VI modification d'un EPS

On transforme par électroporation la souche *S. thermophilus* CNCM I-1422, déposée le 18 mai 1994, par le plasmide pSA3 recombinant de l'exemple V. Cette souche Gram-positive présente au microscope un aspect de coques non flagellées formant des chaînettes, elle ne fait pas de spores, elle est anaéorobe facultative, et elle produit un EPS ayant la composition Glc:Gal=2:2.

### Exemple VII modification d'un EPS

On transforme par électroporation la souche *S. thermophilus* CNCM I-1351, déposée le 5 août 1993, par le plasmide pSA3 recombinant de l'exemple V. Cette souche Gram-positive présente au microscope un aspect de coques non flagellées formant des chaînettes, elle ne fait pas de spores, elle est anaéorobe facultative, et elle produit un EPS ayant la composition Glc:Gal:Rha=1:3:2

### Exemple VIII modification d'un EPS

On isole de l'ADN chromosomique de la souche CNCM I-1590 par le méthode de Slos *et al.* (Appl. Environ. Microbiol., 57, 1333-1339, 1991). On digère la préparation d'ADN par *Sac*I et *Bam*HI, on sépare les fragments d'ADN par électrophorèse sur gel d'agarose 0,7%, on élue les fragments de 12 à 16kb, on ligue l'ADN extrait au vecteur pJIM2279 (obtenu de P. Renault, INRA, Jouy-en-Josas, Paris, France) préalablement digéré par *Sac*I et *Bam*HI puis déphosphorylé. On transforme la souche *Lactococcus lactis* MG1363 (J. Bacteriol., 154, 1-9, 1983), cultivée sur milieu GM17 à 30°C, par la méthode de De Vos *et al.* (Gene, 85, 169-176, 1989). On sélectionne les clones transformés par hybridation du DNA génomique des clones avec l'une des sondes ayant la séquence SEQ ID NO:15, 16, 17, 18 et 19. Parmi 400 transformants, 6 clones positifs sont sélectionnés, dont 1 comprend un plasmide appelé pFS101 représenté à la figure 1.C.

Pour déterminer si le plasmide pFS101 est capable d'induire la production d'EPS recombinant, *L. lactis* MG1363 est retransformé par pFS101, et directement étalé sur le milieu solide rouge de ruthénium. A titre de comparaison, *L. lactis* MG1363 est transformé par le plasmide pJIM2279 puis est directement étalé sur le milieu solide rouge de ruthénium Les résultats montrent que toutes les colonies comprenant pJIM2279 ont un phénotype rouge (3000 colonies EPS(-)), tandis que plus de 99,5% des colonies comprenant pFS101 ont un phénotype blanc (800 colonies EPS(+), à l'exception de 2 colonies). La souche *L*. *lactis* MG1363 transformé par pFS101 produit donc un EPS recombinant.

On fait produire l'EPS de la souche *L. lactis* MG1363 transformée par pFS101, en la cultivant dans le milieu MAM, à un pH de 5,5, à 30°C sous agitation magnétique de 60 rotation par minute. On isole l'EPS recombinant en mélangeant le milieu de culture à 40% d'acide trichloro-acétique, en centrifugeant le mélange 20 min à 8000g, en mélangeant un volume égal d'acétone au précipité, en incubant le tout à 4°C pendant 12h, en précipitant le mélange à 10000g pendant 1h, en mettant en suspension le précipité dans de l'eau, en ajustant le pH du mélange à 7, en le dialysant contre de l'eau pendant 24h, en l'ultracentrifugeant à 100000g pendant 1h, en récupérant le surnageant, puis en lyophilisant le surnageant. A titre de comparaison, on cultive la souche *L. lactis* MG1363 transformée par pJIM2279 dans les mêmes conditions et on isole les sucres de la même manière.

On détermine la quantité de sucres neutres totaux par la méthode de Dubois *et al.* (Anal. Chem., 28, 350-356, 1956). Les résultats montrent que la souche transformée par pFS101 produit 10mg/l de sucres, exprimé en glucose équivalent, tandis la souche transformée par pJIM2279 produit des traces de sucre (< 1mg/l).

On estime le poids moléclaire de l'EPS recombinant par chromatographie sur une colonne de gel-filtration Superose-6 (Pharmacia) qui est connectée au système FPLC (Pharmacia) préalablement calibré avec du dextran commercial (Sigma) de 2x10⁶ à 5x10³ Dalton (Da). Pour cela, on dépose sur la colonne 0,25 à 1ml d'un échantillon comprenant 250µg de sucres neutres, on l'élue par un flux de 0,5ml/min dans un tampon phophate 50mM pH7,2. Pour comparaison, de la même manière on sépare les sucres produits par la souche transformée par pJIM2279. Les résultats présentés à la figure 2 montrent que la souche transformée par pJIM2279 produit une petite quantité de polysaccharides hétérogènes ayant certainement pour origine la paroi cellulaire (2-0,5x10⁶ Da; fractions 8-15) et une grande quantité d'oligosaccharides de petits poids moléculaires (mono- et di-saccharides; fractions 20-22). Par contre, la souche transformée par pFS101 présente manifestement un EPS recombinant de haut poids moléculaire d'environ 2x10⁶ Da (fraction 9).

On détermine la composition en sucres de l'EPS recombinant par chromatographie en phase gazeuse par la méthode de Neeser *et al*. (Anal. Biochem., 142, 58-67, 1984). Les résultats montrent que le milieu de culture de la souche transformée par pFS101 comprend en molarité un ratio 1:3 de Glc:Gal. On peut détecter des traces de rhamnose issues de la paroi cellulaire. Par contre, on ne détecte pas de GalNac.

La composition de l'EPS produit par la souche *L. lactis* MG1363 transformée par pFS101 est donc différente de celle de l'EPS produit par la souche *S. thermophilus* CNCM I-1590. On peut raisonnablement estimer que la structure de l'EPS recombinant est la même que celle de l'EPS de la souche CNCM I-1590, à la différence près que le GalNac est remplacé par un galactose.

## Revendications

1. Molécule d'ADN codant pour au moins une enzyme impliquée dans la biosynthèse d'un exopolysaccharide produit par une bactérie lactique, qui est constitué par l'assemblage de plusieurs sucres différents formant une unité répétitive présentant la structure répétée 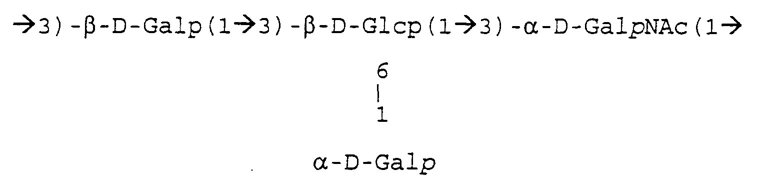 caractérisée en ce qu'elle consiste en un gène choisi dans le groupe de gènes (1) qui sont délimités dans la séquence nucléique SEQ ID NO 1 par les nucléotides 352-1803, 1807-2535, 2547-3239, 3249-3995, 4051-4731, 4898-5854, 6425-7540, 7736-8212, 8221-9192, 9285-10364, 10392-11339, 11302-12222 et 12233-13651, des séquences qui possèdent la même fonction et qui ont un taux d'identité de plus de 70% avec les séquences délimitées dans la séquence nucléotidique SEQ ID NO 1 par les nucléotides 352-1803, 1807-2535, 2547-3239, 3249-3995, 4051-4731, 4898-5854, 6425-7540, 7736-8212, 8221-9192, 9285-10364, 10392-11339, 11302-12222 et 12233-13651, et les séquences nucléotidiques qui possèdent la même fonction et qui s'hybrident dans des conditions stringentes avec les séquences délimitées dans la séquence nucléotidique SEQ ID NO 1 par les nucléotides 352-1803, 1807-2535, 2547-3239, 3249-3995, 4051-4731, 4898-5854, 6425-7540, 7736-8212, 8221-9192, 9285-10364, 10392-11339, 11302-12222 et 12233-13651.

2. Vecteur recombinant comprenant la molécule d'ADN selon la revendication 1.

3. Protéine susceptible d'être impliquée dans la biosynthèse d'un exopolysaccharide produit par une bactérie lactique, qui est constitué par l'assemblage de plusieurs sucres différents formant une unité répétitive présentant la structure répétée 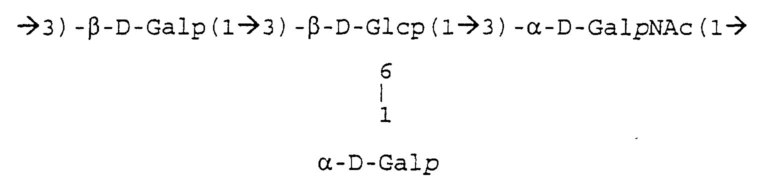 caractérisée en ce qu'elle présente la séquence en acides aminés choisie dans le groupe constitué par les séquences SEQ ID NO 2, SEQ ID NO 3, SEQ ID NO 4, SEQ ID NO 5, SEQ ID NO 6, SEQ ID NO 7, SEQ ID NO 8, SEQ ID NO 9, SEQ ID NO 10, SEQ ID NO 11, SEQ ID NO 12, SEQ ID NO 13, SEQ ID NO 14 et les séquences qui possèdent la même fonction et qui ont un taux d'identité de plus de 70% avec les séquences SEQ ID NO 2, SEQ ID NO 3, SEQ ID NO 4, SEQ ID NO 5, SEQ ID NO 6, SEQ ID NO 7, SEQ ID NO 8, SEQ ID NO 9, SEQ ID NO 10, SEQ ID NO 11, SEQ ID NO 12, SEQ ID NO 13, SEQ ID NO 14.

4. Bactérie lactique transformée par le vecteur de la revendication 2.

5. Procédé de production d'un exopolysaccharide produit par une bactérie lactique, qui est constitué par l'assemblage de plusieurs sucres différents formant une unité répétitive, dans lequel :
- on clone dans un vecteur une molécule d'ADN codant pour les enzymes impliquées dans la biosynthèse de l'exopolysaccharide selon la revendication 3, ledit vecteur comprenant en outre une séquence permettant la réplication autonome ou l'intégration dans une cellule hôte,
- on transforme une cellule hôte par ledit vecteur,
- puis on cultive la cellule hôte transformée dans des conditions appropriées pour la production dudit exopolysaccharide.

6. Procédé selon la revendication 5, dans lequel le vecteur comprend en outre des séquences promotrice et d'activation traductionnelle fonctionnelles dans ladite cellule hôte.

7. Procédé de production d'un exopolysaccharide produit par une bactérie lactique, qui est constitué par l'assemblage de plusieurs sucres différents formant une unité répétitive, dans lequel :
- on clone dans un vecteur une molécule d'ADN codant pour au moins une des enzymes impliquées dans la biosynthèse de l'exopolysaccharide selon la revendication 3,
- on transforme par ledit vecteur une bactérie lactique produisant le cas échéant un autre exopolysaccharide,
- puis on cultive la bactérie lactique transformée dans des conditions appropriées pour la production d'un nouvel exopolysaccharide.

8. Procédé selon la revendication 6 ou 7, dans lequel on clone dans un vecteur une molécule d'ADN selon la revendication 1.

9. Utilisation d'un fragment d'ADN de la séquence SEQ ID NO 1 ou de son brin complémentaire, d'au moins 15 paires de base, comme amorce utilisable dans une réaction de PCR ou comme sonde pour détecter *in vitro* ou inactiver *in vivo* des gènes de bactéries lactiques impliquées dans la biosynthèse d'un exopolysaccharide.

## Claims

1. DNA molecule encoding at least one enzyme involved in the biosynthesis of an exopolysaccharide being produced by a lactic bacterium, which is formed by an assembly of various different sugars forming a repetitive unit having the following structure : 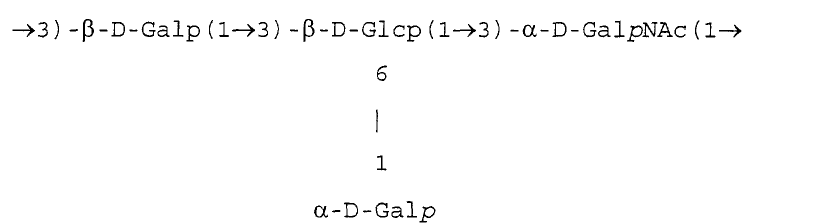 characterized in that it consists in a gene selected from the group of genes (1) which are limited within the nucleic sequence SEQ ID NO 1 by nucleotides 352-1803, 1807-2535, 2547-3239, 3249-3995, 4051-4731, 4898-5854, 6425-7540, 7736-8212, 8221-9192, 9285-10364, 10392-11339, 11302-12222 and 12233-13651, sequences which have the same function and an identity rate of more than 70% with the sequences being limited within the nucleotidic sequence SEQ ID NO 1 by nucleotides 352-1803, 1807-2535, 2547-3239, 3249-3995, 4051-4731, 4898-5854, 6425-7540, 7736-8212, 8221-9192, 9285-10364, 10392-11339, 11302-12222 et 12233-13651, and the nucleotidic sequences which have the same function and are hybridised in stringent conditions with the sequences being limited within the nucleotidic sequence SEQ ID NO 1 by nucleotides 352-1803, 1807-2535, 2547-3239, 3249-3995, 4051-4731, 4898-5854, 6425-7540, 7736-8212, 8221-9192, 9285-10364, 10392-11339, 11302-12222 et 12233-13651.

2. Recombinant vector comprising the DNA molecule according to claim 1.

3. Protein suitable to be involved in the biosynthesis of an exopolysaccharide being produced by a lactic bacterium, which is formed by an assembly of various different sugars forming a repetitive unit having the following structure : 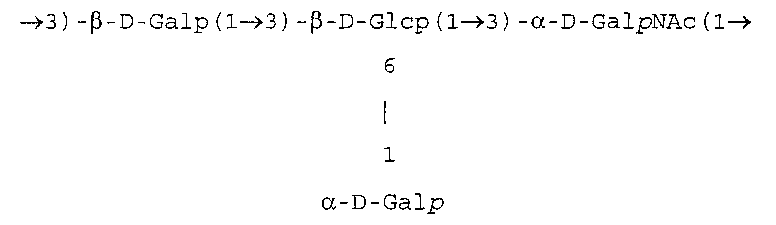 characterized in that it has the amino-acid sequence selected from the group consisted in sequences SEQ ID NO 2, SEQ ID NO 3, SEQ ID NO 4, SEQ ID NO 5, SEQ ID NO 6, SEQ ID NO 7, SEQ ID NO 8, SEQ ID NO 9, SEQ ID NO 10, SEQ ID NO 11, SEQ ID NO 12, SEQ ID NO 13, SEQ ID NO 14 and sequences which have the same function and an identity rate of more than 70% with sequences SEQ ID NO 2, SEQ ID NO 3, SEQ ID NO 4, SEQ ID NO 5, SEQ ID NO 6, SEQ ID NO 7, SEQ ID NO 8, SEQ ID NO 9, SEQ ID NO 10, SEQ ID NO 11, SEQ ID NO 12, SEQ ID NO 13, SEQ ID NO 14.

4. Lactic bacterium transformed by the vector according to claim 2.

5. Process for producing an exopolysaccharide being produced by a lactic bacterium, which is formed by an assembly of various different sugars forming a repetitive unit, said process consisting in :
- cloning into a vector a DNA molecule encoding enzymes involved in the biosynthesis of the exopolysaccharide according to claim 3, said vector comprising further a sequence allowing for the autonomous replication or the integration into an host cell,
- transforming an host cell through said vector, and
- then culturing the transformed host cell in convenient conditions for producing said exopolysaccharide.

6. Process according to claim 5, wherein the vector comprises additionally functional promotor and translation activator sequences in said host cell.

7. Process for producing an exopolysaccharide being produced by a lactic bacterium, which is formed by an assembly of various different sugars forming a repetitive unit, said process consisting in :
- cloning into a vector a DNA molecule encoding at least one of the enzymes involved in the biosynthesis of the exopolysaccharide according to claim 3,
- transforming through said vector a lactic bacterium producing the case being another exopolysaccharide, and
- then culturing the transformed lactic bacterium in convenient conditions for producing a new exopolysaccharide.

8. Process according to claim 6 or 7, wherein a DNA molecule according to claim 1 is cloned into a vector.

9. Use of a DNA fragment of the sequence SEQ ID NO 1 or the complementary strand thereof, of at least 15 base pairs, as an initiator useful in a PCR reaction or as a probe so as to detect *in vitro* or inactivate *in vivo* genes from lactic bacteria involved in the biosynthesis of an exopolysaccharide.

## Patentansprüche

1. DNA-Molekül, das für mindestens ein Enzym kodiert, das an der Biosynthese eines durch eine Milchsäurebakterie erzeugten Exopolysaccharids beteiligt ist, das gebildet wird durch Aneinanderfügen mehrerer verschiedener Zucker, die eine repetitive Einheit bilden, welche die sich wiederholende Struktur 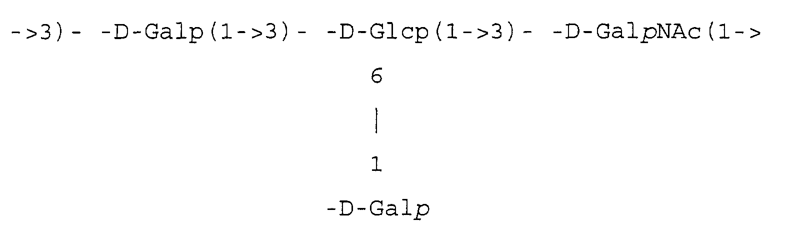 aufweist, dadurch gekennzeichnet, daß es aus einem Gen besteht, das in der Gruppe von Genen (1) gewählt ist, die in der Nukleotidsequenz SEQ ID NO 1 begrenzt sind durch die Nukleotide 352-1803, 1807-2535, 2547-3239, 3249-3995, 4051-4731, 4898-5854, 6425-7540, 7736-8212, 8221-9192, 9285-10364, 10392-11339, 11302-12222 und 12233-13651, Sequenzen, die die gleiche Funktion haben, und die einen Identitätsgrad von mehr als 70% mit den Sequenzen haben, die in der Nukleotidsequenz SEQ ID NO 1 durch die Nukleotide 352-1803, 1807-2535, 2547-3239, 3249-3995, 4051-4731, 4898-5854, 6425-7540, 7736-8212, 8221-9192, 9285-10364, 10392-11339, 11302-12222 und 12233-13651 begrenzt sind, und die Nukleotid-sequenzen, die die gleiche Funktion haben, und die unter harten Bedingungen mit den Sequenzen hybridisieren, die in der Nukleotidsequenz SEQ ID NO 1 durch die Nukleotide 352-1803, 1807-2535, 2547-3239, 3249-3995, 4051-4731, 4898-5854, 6425-7540, 7736-8212, 8221-9192, 9285-10364, 10392-11339, 11302-12222 und 12233-13651 begrenzt sind.

2. Rekombinanter Vektor, der das DNA-Molekül gemäß dem Anspruch 1 aufweist.

3. Protein, das geeignet ist, an der Biosynthese eines von einer Milchsäurebakterie erzeugten Exopolysaccharids beteiligt zu sein, und das gebildet wird durch Aneinanderfügen mehrerer verschiedener Zucker, die eine repetitive Einheit bilden, welche die sich wiederholende Struktur 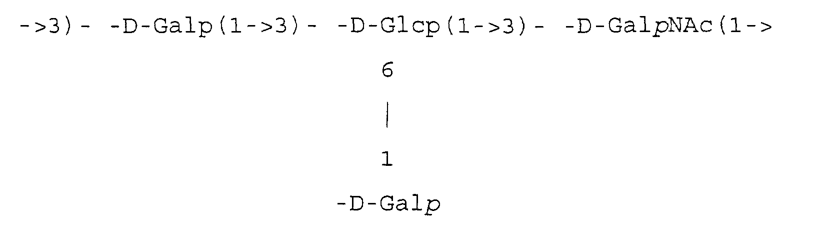 aufweist, dadurch gekennzeichnet, daß es die Aminosäuresequenz aufweist, die in der Gruppe gewählt ist, die besteht aus den Sequenzen SEQ ID NO 2, SEQ ID NO 3, SEQ ID NO 4, SEQ ID NO 5, SEQ ID NO 6, SEQ ID NO 7, SEQ ID NO 8, SEQ ID NO 9, SEQ ID NO 10, SEQ ID NO 11, SEQ ID NO 12, SEQ ID NO 13, SEQ ID NO 14, und den Sequenzen, die die gleiche Funktion haben, und die einen Identitätsgrad von mehr als 70% mit den Sequenzen SEQ ID NO 2, SEQ ID NO 3, SEQ ID NO 4, SEQ ID NO 5, SEQ ID NO 6, SEQ ID NO 7, SEQ ID NO 8, SEQ ID NO 9, SEQ ID NO 10, SEQ ID NO 11, SEQ ID NO 12, SEQ ID NO 13 und SEQ ID NO 14 haben.

4. Milchsäurebakterie, die durch den Vektor des Anspruchs 2 transformiert wurde.

5. Verfahren zur Herstellung eines von einer Milchsäurebakterie erzeugten Exopolysaccharids, das gebildet wird durch Aneinanderfügen mehrerer verschiedener Zucker, die eine repetitive Einheit bilden, wobei bei dem Verfahren:
- in einem Vektor ein DNA-Molekül kloniert wird, das für die Enzyme kodiert, die an der Biosynthese des Exopolysaccharids gemäß dem Anspruch 3 beteiligt sind, wobei der Vektor außerdem eine Sequenz aufweist, welche die autonome Replikation oder die Integration in eine Wirtszelle ermöglicht,
- eine Wirtszelle durch diesen Vektor transformiert wird, und
- dann die transformierte Wirtszelle unter für die Herstellung des Exopolysaccharids geeigneten Bedingungen gezüchtet wird.

6. Verfahren gemäß Anspruch 5, bei dem der Vektor außerdem funktionale Promotor- und Translationsaktivierungs-Sequenzen in der Wirtszelle aufweist.

7. Verfahren zur Herstellung eines von einer Milchsäurebakterie erzeugten Exopolysaccharids, das gebildet wird durch Aneinanderfügen mehrerer verschiedener Zucker, die eine repetitive Einheit bilden, wobei bei dem Verfahren:
- in einem Vektor ein DNA-Molekül kloniert wird, das für mindestens eines der Enzyme kodiert, die an der Biosynthese des Exopolysaccharids gemäß dem Anspruch 3 beteiligt sind,
- durch diesen Vektor eine Milchsäurebakterie transformiert wird, die gegebenenfalls ein anderes Exopolysaccharid erzeugt, und
- dann die transformierte Milchsäurebakterie unter für die Herstellung eines neuen Exopolysaccharids geeigneten Bedingungen gezüchtet wird.

8. Verfahren gemäß Anspruch 6 oder 7, bei dem ein DNA-Molekül gemäß dem Anspruch 1 in einem Vektor kloniert wird.

9. Verwendung eines DNA-Fragments der Sequenz SEQ ID NO 1 oder ihres komplementären Strangs mit mindestens 15 Basenpaaren als bei einer PCR-Reaktion verwendbarer Primer, oder als Sonde, um Gene von Milchsäurebakterien, die an der Biosynthese eines Exopolysaccharids beteiligt sind, *in vitro* nachzuweisen oder *in vivo zu* inaktivieren.
